# EUROPEAN PATENT SPECIFICATION

(11) **EP 1 283 830 B1**
(45) Date of publication and mention of the grant of the patent: **18.06.2008**
(21) Application number: 01943302.8
(22) Date of filing: 30.04.2001
(51) Int. Cl.: C07D 277/46, C07D 277/56, C07D 213/82, C07D 333/24, A61K 31/426, A61K 31/427, A61K 31/4436, A61P 3/10

(54) **PARA-AMINE SUBSTITUTED PHENYLAMIDE GLUCOKINASE ACTIVATORS**
PARA-AMIN SUBSTITUIERTE PHENYLAMID GLUKOKINASE ACTIVATOREN
ACTIVATEURS DE GLUCOKINASE DE TYPE PHENYLAMIDES SUBSTITUES PARA-AMINE

(30) Priority: 08.05.2000 US 202389 P
(43) Date of publication of application: 19.02.2003
(73) Proprietor: F. Hoffmann-La Roche AG, 4070 Basel (CH)
(72) Inventor: BIZZARRO, Fred, Thomas, Colonia, NJ 07067 (US); HAYNES, Nancy-Ellen, Cranford, NJ 07016 (US); SARABU, Ramakanth, Towaco, NJ 07082 (US)
(74) Representative: Brodbeck, Michel
(86) International application number: PCT/EP2001/004859
(87) International publication number: WO 2001/085707

(56) References cited:
- US-A- 5 599 826
- US-A- 6 011 048

## Description

Glucokinase (GK) is one of four hexokinases found in mammals [Colowick, S.P., in The Enzymes, Vol. 9 (P. Boyer, ed.) Academic Press, New York, NY, pages 1-48, 1973]. The hexokinases catalyze the first step in the metabolism of glucose, i.e., the conversion of glucose to glucose-6-phosphate. Glucokinase has a limited cellular distribution, being found principally in pancreatic β-cells and liver parenchymal cells. In addition, GK is a rate-controlling enzyme for glucose metabolism in these two cell types that are known to play critical roles in whole-body glucose homeostasis [Chipkin, S.R., Kelly, K.L., and Ruderman, N.B. in Joslin's Diabetes (C.R. Khan and G.C. Wier, eds.), Lea and Febiger, Philadelphia, PA, pages 97-115, 1994]. The concentration of glucose at which GK demonstrates half-maximal activity is approximately 8 mM. The other three hexokinases are saturated with glucose at much lower concentrations (<1 mM). Therefore, the flux of glucose through the GK pathway rises as the concentration of glucose in the blood increases from fasting (5 mM) to postprandial (=10-15 mM) levels following a carbohydrate-containing meal [Printz, R.G., Magnuson, M.A., and Granner, D.K. in Ann. Rev. Nutrition Vol. 13 (R.E. Olson, D.M. Bier, and D.B. McCormick, eds.), Annual Review, Inc., Palo Alto, CA, pages 463-496, 1993]. These findings contributed over a decade ago to the hypothesis that GK functions as a glucose sensor in β-cells and hepatocytes (Meglasson, M.D. and Matschinsky, F.M. Amer. J. Physiol. 246, E1-E13, 1984). In recent years, studies in transgenic animals have confirmed that GK does indeed play a critical role in whole-body glucose homeostasis. Animals that do not express GK die within days of birth with severe diabetes while animals overexpressing GK have improved glucose tolerance (Grupe, A., Hultgren, B., Ryan, A. et al., Cell 83, 69-78, 1995; Ferrie, T., Riu, E., Bosch, F. et al., FASEB J., 10, 1213-1218, 1996). An increase in glucose exposure is coupled through GK in β-cells to increased insulin secretion and in hepatocytes to increased glycogen deposition and perhaps decreased glucose production.

The finding that type II maturity-onset diabetes of the young (MODY-2) is caused by loss of function mutations in the GK gene suggests that GK also functions as a glucose sensor in humans (Liang, Y., Kesavan, P., Wang, L. et al., Biochem. J. 309, 167-173, 1995). Additional evidence supporting an important role for GK in the regulation of glucose metabolism in humans was provided by the identification of patients that express a mutant form of GK with increased enzymatic activity. These patients exhibit a fasting hypoglycemia associated with an inappropriately elevated level of plasma insulin (Glaser, B., Kesavan, P., Heyman, M. et al., New England J. Med. 338, 226-230, 1998). While mutations of the GK gene are not found in the majority of patients with type II diabetes, compounds that activate GK and, thereby, increase the sensitivity of the GK sensor system will still be useful in the treatment of the hyperglycemia characteristic of all type IT diabetes. Glucokinase activators will increase the flux of glucose metabolism in β-cells and hepatocytes, which will be coupled to increased insulin secretion. Such agents would be useful for treating type II diabetes.

This invention provides an amide selected from the group consisting of a compound of the formula: 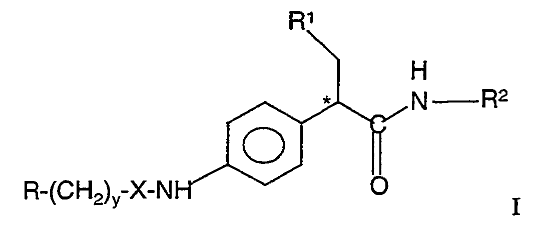 wherein X is 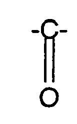 or 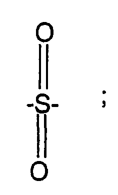 R is perfluoro-lower alkyl, lower alkyl, 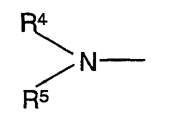 lower alkoxycarbonyl, a heteroaromatic ring, connected by a ring carbon atom, containing from 5 to 6 ring members with from 1 to 3 heteroatoms selected from the group consisting of oxygen, sulfur and nitrogen, unsubstituted aryl containing 6 or 10 ring carbon atoms, a nitro or a lower alkyl substituted aryl, which aryl contains 6 or 10 ring carbon atoms, a saturated 5- to 6-membered cycloheteroalkyl ring, connected by a ring carbon atom, containing 1 or 2 heteroatoms selected from the group consisting of oxygen, sulfur and nitrogen, or a cycloalkyl ring having 5 or 6 carbon atoms; R¹ is a cycloalkyl having 5 or 6 carbon atoms; R² is a five- or six-membered heteroaromatic ring connected by a ring carbon atom to the amide group in the remainder of the compound, which heteroaromatic ring contains from 1 to 3 heteroatoms selected from the group consisting of oxygen, sulfur and nitrogen with a first heteroatom being nitrogen adjacent to the connecting ring carbon atom, said heteroaromatic ring being unsubstituted or monosubstituted at a position on a ring carbon atom other than adjacent to said connecting carbon atom with a substituent selected from the group consisting of lower alkyl, or 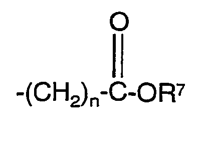 n and y independently are an integer of from () to 4, R⁴, R⁵ and R⁷ are independently hydrogen or lower alkyl, * denotes the asymmetric carbon atom; and wherein "lower" means 1 to 7 carbon atoms;
and a pharmaceutically acceptable salt thereof.

The compounds of formula I are glucokinase activators useful for increasing insulin secretion in the treatment of type II diabetes.

The present invention also relates to a pharmaceutical composition comprising a compound of formula I and a pharmaceutically acceptable carrier and/or adjuvant. Furthermore, the present invention relates to the use of such compounds as therapeutic active substances as well as to their use for the preparation of medicaments for the treatment or prophylaxis of type II diabetes. The present invention further relates to processes for the preparation of the compounds of formula I.

The compounds of formula I have the following embodiments 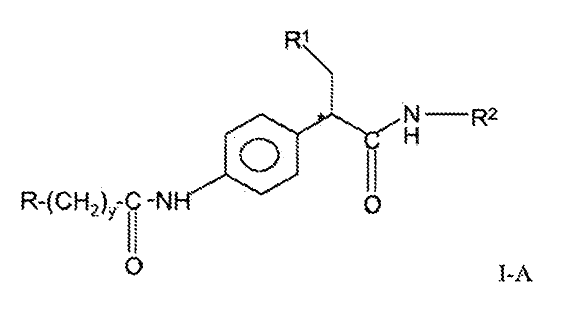 and 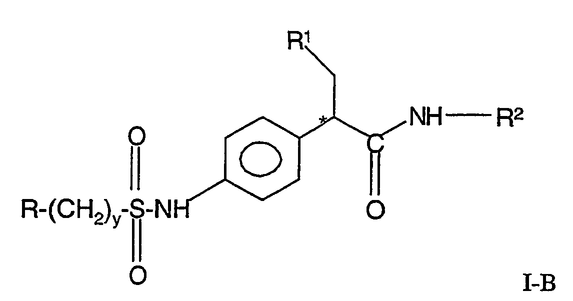 wherein R, R¹, R², * and y are as above;

In the compound of formulae I, IA and IB, the "*" designates that the asymmetric carbon atom in the compounds with the R optical configuration being preferred. The compounds of formula I may be present in the R or as a racemic or other mixtures of compounds having the R and S optical configuration at the asymmetric carbon shown. The pure R enantiomers are preferred.

As used throughout this application, the term "lower alkyl" includes both straight chain and branched chain alkyl groups having from 1 to 7 carbon atoms, such as methyl, ethyl, propyl, isopropyl, preferably methyl and ethyl. As used herein, the term "halogen or halo" unless otherwise stated, designates all four halogens, i.e. fluorine, chlorine, bromine and iodine.

As used herein, perfluoro-lower alkyl means any lower alkyl group wherein all of the hydrogens of the lower alkyl group are substituted or replaced by fluoro. Among the preferred perfluoro-lower alkyl groups are trifluoromethyl, pentafluoroethyl, heptafluoropropyl etc., with trifluoromethyl being especially preferred.

As used herein, the term "aryl" signifies "polynuclear" and mononuclear unsubstituted aromatic hydrocarbon groups such as phenyl, naphthy containing either 6 or 10 carbon atoms which aryl groups in the compounds of formulae I, IA and IB are either phenyl and naphthyl. The aryl substituent can be unsubstituted or substituted, preferably monosubstituted with a nitro or lower alkyl substituted.

R can be any five- or six-membered saturated cycloheteroalkyl ring containing from 1 to 2 heteroatoms selected from the group consisting of sulfur, oxygen or nitrogen. Any such five- or six-membered saturated heterocyclic ring can be used in accordance with this invention. Among the preferred rings are morpholinyl, pyrrolidinyl, piperazinyl, piperidinyl, etc. When R is a saturated cyclic heteroacetyl ring, it is connected to the remainder of the molecule of formula I through a ring carbon atom.
The heteroaromatic ring defined by R and R² can be five- or six-membered heteroaromatic ring having from 1 to 3 heteroatoms selected from the group consisting of oxygen, nitrogen, and sulfur which is connected by a ring carbon to the remainder of the molecule as shown. The heteroaromatic ring defined by R² contains a first nitrogen heteroatom adjacent to the connecting ring carbon atom and if present, the other heteroatoms can be oxygen, sulfur, or nitrogen. Among the preferred heteroaromatic rings include pyridinyl, pyrimidinyl and thiazolyl. These heteroaromatic rings which constitute R² are connected via a ring carbon atom to the amide group to form the amides of formula I. The ring carbon atom of the heteroaromatic ring which is connected to the amide to form the compound of formula I does not contain any substituent. When R² is an unsubstituted or mono-substituted five- or six-membered heteroaromatic ring, the rings contain a nitrogen heteroatom adjacent to the connecting ring carbon.

In one embodiment of the present invention, R² is a five-membered heteroaromatic ring, such as thiazolyl, connected by a ring carbon atom to the amide group to the remainder of the compound, which heteroaromatic ring contains from 1 to 3 heteroatoms selected from the group consisting of oxygen, sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent selected from lower alkyl and -(CH₂)ₙ-C(O)OR⁷; and n and R⁷ are as defined above.

In another embodiment of the present invention, R² is a six-membered heteroaromatic ring, such as pyridyl, connected by a ring carbon atom to the amide group to the remainder of the compound, which heteroaromatic ring contains from 1 to 3 heteroatoms selected from the group consisting of oxygen, sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent selected from lower alkyl and -(CH₂)ₙ-C(O)OR⁷; and n and R⁷ are as defined above.

Preferable substituent R¹ in accordance with the present invention is cyclopentyl.

In one embodiment of the present invention, substituent R is phenyl, naphthyl or nitro substituted phenyl or naphthyl, preferably nitro substituted phenyl. In another embodiment of the present invention, R is unsubstituted phenyl. In still another embodiment of the present invention, R is a heteroaromatic ring, connected by a ring carbon atom, containing from 5 to 6 ring members with from 1 to 3 heteroatoms selected from the group consisting of oxygen, sulfur and nitrogen, with thienyl and pyridyl being especially preferred. In still another embodiment of the present invention, R is lower alkoxy carbonyl. In still another embodiment of the present invention, R is lower alkyl or perfluoro lower alkyl. In still another embodiment of the present invention, R is -N(R⁴,R⁵) and R⁴ and R⁵ are as defined in claim 1.

In a preferable embodiment of the present invention, n and y are independently 0 or 1. Preferred R⁴ and R⁵ are lower alkyl.

The term "pharmaceutically acceptable salts" as used herein include any salt with both inorganic or organic pharmaceutically acceptable acids such as hydrochloric acid, hydrobromic acid, nitric acid, sulfuric acid, phosphoric acid, citric acid, formic acid, maleic acid, acetic acid, succinic acid, tartaric acid, methanesulfonic acid, para-toluene sulfonic acid and the like. The term "pharmaceutically acceptable salts" also includes any pharmaceutically acceptable base salt such as amine salts, trialkyl amine salts and the like. Such salts can be formed quite readily by those skilled in the art using standard techniques.

During the course of the reaction the various functional groups such as the free carboxylic acid will be protected via conventional hydrolyzable ester protecting groups. As used herein, the term "hydrolyzable ester" designates any ester conventionally used for protecting carboxylic acids which can be hydrolyzed to yield the respective carboxyl group. Exemplary ester groups useful for those purposes are those in which the acyl moieties are derived from a lower alkanoic, aryl lower alkanoic, or lower alkane dicarboxylic acid. Among the activated acids which can be utilized to form such groups are acid anhydrides, acid halides, preferably acid chlorides or acid bromides derived from aryl or lower alkanoic acids. Example of anhydrides are anhydrides derived from monocarboxylic acid such as acetic anhydride, benzoic acid anhydride, and lower alkane dicarboxylic acid anhydrides, e.g. succinic anhydride as well as chloro formates e.g. trichloro, ethylchloro formate being preferred.

Among the embodiments of the amides of formula I-A are those compounds where R¹ is cyclopentyl [compounds of formula I-A1]. The embodiments of the compounds of formula I-A1 are those compounds where R² is a 5-membered heteroaromatic ring, preferably thiazolyl. Among the embodiment of compounds of formula I-A1 are those compounds where R² is a 5-membered heteroaromatic ring are those compounds where R is:
a) aryl, preferably phenyl;
b) aryl substituted with a nitro group, preferably nitro substituted phenyl;
c) heteroaromatic ring such as pyrimidinyl, thiazolyl and pyridinyl; or
d) lower alkoxy carbonyl.

Among other embodiments of the compounds of formula I-A1 are those compounds where R² is a substituted or unsubstituted 6-membered heteroaromatic ring such as pyridinyl. Among the embodiments of compounds of formula I-A1 where R² is a substituted or unsubstituted 6-membered heteroaromatic ring are those compounds where:
a) R is an unsubstituted aryl or a heteroaromatic ring, particularly pyridinyl;
b) R is lower alkoxy carbonyl; or
c) R is perfluoro-lower alkyl.

Among the embodiments of the compounds of formula I-B are those compounds wherein R¹ is cyclopentyl [compounds of formula I-B1]. Among the embodiments of compounds of formula I-B 1 are those compounds where R² is a 5-membered heteroaromatic ring, preferably unsubstituted or substituted thiazolyl, with preferred embodiments being those compounds where R is a
a) nitro substituted aryl such as nitro substituted phenyl;
b) aryl such as phenyl;
c) lower alkyl;
d) perfluoro-lower alkyl; or
e) 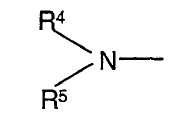 where R⁴ and R⁵ are as above.

Preferred compounds in accordance with the present invention are those amides of above formula I, wherein X is -C(O)- or -S(O)₂-; R is perfluoro-lower alkyl, lower alkyl, -N(R⁴,R⁵), lower alkoxycarbonyl, a heteroaromatic ring, connected by a ring carbon atom, containing from 5 to 6 ring members with 1 heteroatom selected from sulfur and nitrogen, phenyl, a nitro substituted phenyl; R¹ is cyclopentyl; R² is a five- or six-membered heteroaromatic ring connected by a ring carbon atom to the amide group to the remainder of the compound, which heteroaromatic ring contains 1 or 2 heteroatoms selected from sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent -(CH₂)ₙ-C(O)-OR⁷; n and y are independently 0 or 1; R⁴ and R⁵ are lower alkyl; and R⁷ is hydrogen or lower alkyl.
Most preferred compounds in accordance with the present invention are:
N-{4-[2-cyclopentyl-1-(thiazol-2-ylcarbamoyl)-ethyl]-phenyl}-4-nitrobenzamide,
N-{4-[2-cyclopentyl-1-(thiazol-2-ylcarbamoyl)-ethyl]-phenyl}-benzamide,
3-cyclopentyl-N-thiazol-2-yl-2-[4-(2-thiophen-2-yl-acetylamino)-phenyl]-propionamide,
N-{4-[2-cyclopentyl-1-(thiazol-2-ylcarbamoyl)-ethyl]-phenyl}-isonicotinamide,
2-(3-cyclopentyl-2-{4-[(pyridine-3-carbonyl)-amino]-phenyl}-propionylamino)-thiazole-4-carboxylic acid ethyl ester,
N- {4-[2-cyclopentyl-1-(thiazol-2-ylcarbamoyl)-ethyl]-phenyl}-nicotinamide,
[2-(3-cyclopentyl-2-{4-[(pyridine-3-carbonyl)-amino]-phenyl}-propionylamino)-thiazol-4-yl]-acetic acid ethyl ester,
N- {4-[2-cyclopentyl-1-(pyridin-2-ylcarbamoyl)-ethyl]-phenyl }-nicotinamide,
6-(3-cyclopentyl-2-{4-[(pyridine-3-carbonyl)-amino]-phenyl}-propionylamino)-nicotinic acid methyl ester,
6-(3-cyclopentyl-2-{4-[(pyridine-3-carbonyl)-amino]-phenyl}-propionylamino)-nicotinic acid,
N-(4-[2-cyaopentyl-1-(thiazol-2-ylcarbamoyl)-ethyl]-phenyl)-oxalamic acid methyl ester,
acetic acid {4-[2-cyclopentyl-l-(thiazol-2-ylcarbamoyl)-ethyl]-phenylcarbamoyl }-methyl ester,
3-cyclopentyl-2-(4-methanesulfonylamino-phenyl)-N-thiazol-2-yl-propionamide,
3-cyclopentyl-N-thiazol-2-yl-2-(4-trifluoromethanesulfonylamino-phenyl)-propionamide,
3-cyclopentyl-N-thiazol-2-yl-2-[4-(2,2,2-trifluoro-ethanesulfonylamino)-phenyl]-propionamide,
N-{ 4-[2-cyclopentyl-1-(thiazol-2-ylcarbamoyl)-ethyl]-phenyl }-dimethylsulfamide, and
3-cyclopentyl-2-[4-(4-nitro-benzenesulfonylamino)-phenyl]-N-thiazol-2-yl-propionamide.

The compounds of formula I which are the compounds of formulae I-A and I-B are both prepared from the compound of the formula: 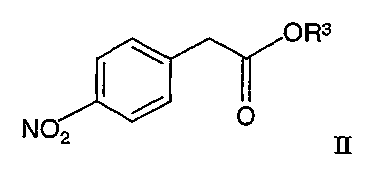 where R³ taken together with its attached oxygen atom forms a hydrolyzable ester protecting group.

In accordance with an embodiment of this invention, the compound of formula II is converted to the compound of formula I-A via the following reaction scheme: 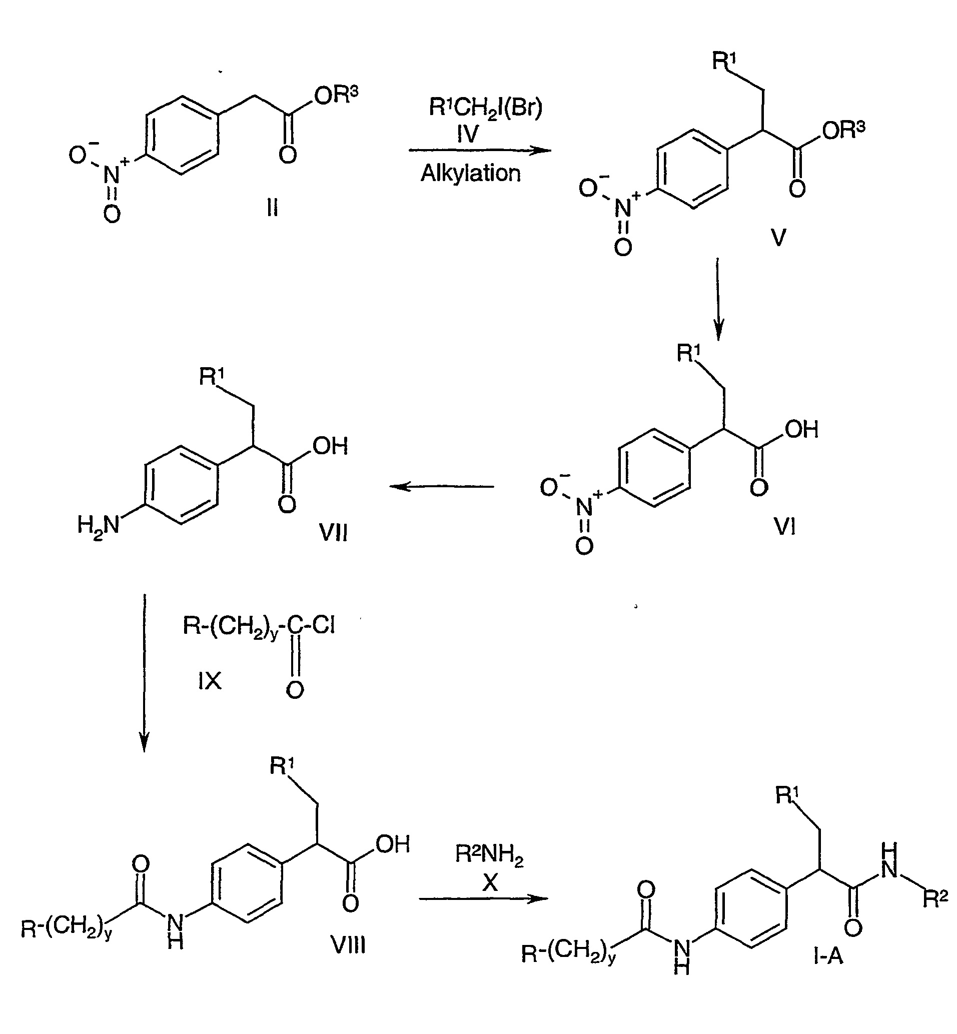 wherein R, R¹, R² and y are as above and R³ taken together with its attached oxygen atom forms a hydrolyzable ester protecting group.

In the first step of this reaction, the compound of formula II is alkylated with the compound of formula IV to form the compound of formula V. Any conventional method of alkylating the alpha carbon atom of an organic acid ester with an alkyl bromide or iodide can be utilized to effect this conversion to produce the compound of formula V. In the next step of this reaction, the compound of formula V is hydrolyzed so as to remove the ester protecting group R³. Any conventional method of ester hydrolysis can be utilized. Among the preferred methods is by treating the compound of formula V with lithium hydroxide in a mixed solvent of water and tetrahydrofuran. On the other hand, sodium hydroxide in methanol or other lower alkanols can be utilized to effect this hydrolysis. The compound of formula VI is converted to the compound of formula VII by reducing the nitro group to an amine group. Any conventional method of reducing a nitro to an amine group can be utilized in carrying out this reaction. Preferably, this reduction can be carried out by treating the compound of formula VI with hydrogen in the presence of a palladium on a carbon catalyst. Any of the conventional conditions for hydrogenation can be utilized in effecting this reduction. Hydrogenation in the presence of a palladium on carbon catalyst will not effect the carboxylic acid group on the compound of formula VI. The compound of formula VII is then converted to the compound of formula VIII by reacting the compound of formula VII with the compound of formula IX to acylate the free amino group. The compound of formula IX is an acid chloride and any conventional method of reacting an acid chloride with a primary amine can be utilized to effect this reaction. The compound of formula VIII is converted to the compound of formula I-A via reaction with the primary amine of formula X. Any conventional method of coupling a carboxylic acid such as the compound of formula VIII with a primary amine such as the compound of formula X produce an amide, i.e., the compound of formula I-A can be utilized to affect this coupling reaction.

The compound of formula I-B can be produced from the compound of formula VI above via the following reaction scheme: 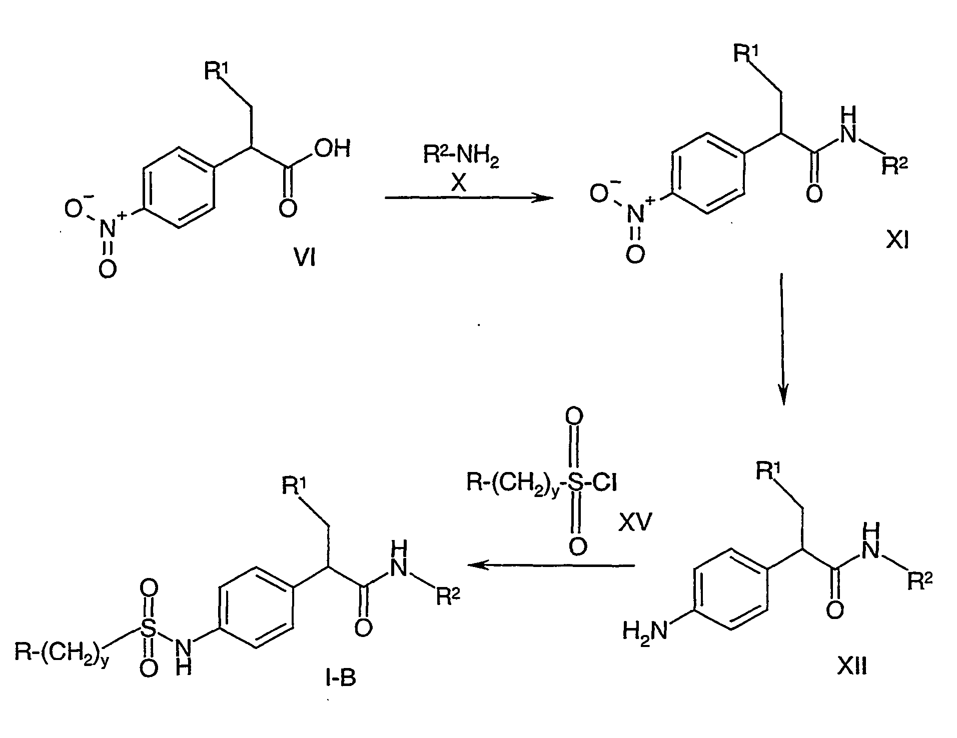 wherein R, R¹, R² and y are as above.

With respect to producing the sulfonamides, the compound of formula VI, as prepared by the aforementioned method, is utilized as a starting material. In this procedure, the compound of formula VI is reacted with the compound of formula X to produce the compound of formula XI. This reaction is carried out in the same manner as set forth with respect to the conversion of the compound of formula VIII to the compound of the formula I-A utilizing any conventional means of amide coupling. In the next step, the compound of formula XI is reduced via a hydrogenation in the presence of a hydrogenation catalyst such as palladium on carbon. This reaction is carried out in the same manner as previously described in connection with the hydrogenation of the compound of formula VI to the compound of formula VII. The compound of formula XII is then reacted with the compound of formula XV to produce the compound of formula I-B. This reaction is carried out by coupling the amino group of the compound of formula XII with the sulfonyl chloride of formula XV to produce the sulfonamides of formula I-B. In carrying out this coupling reaction of a sulfonyl chloride with an amine, any conventional method for forming sulfonamides from sulfonyl chlorides and amines can be utilized. In this manner, the compound of formula I-B is produced.

If it is desired to produce the R enantiomer of the compound of formula I free of the other enantiomer, the compound of formula VI can be separated into this isomer from its racemate by any conventional chemical means. Among the preferred chemical means is to react the compound of formula VI with an optically active base. Any conventional optically active base can be utilized to carry out this resolution. Among the preferred optically active bases are the optically active amine bases such as alpha-methylbenzylamine, quinine, dehydroabietylamine and alpha-methylnaphthylamine. Any of the conventional techniques utilized in resolving organic acids with optically active organic amine bases can be utilized in carrying out this reaction.

In the resolution step, the compound of formula VI is reacted with the optically active base in an inert organic solvent medium to produce salts of the optically active amine with both the R and S isomers of the compound of formula VI. In the formation of these salts, temperatures and pressure are not critical and the salt formation can take place at room temperature and atmospheric pressure. The R and S salts can be separated by any conventional method such as fractional crystallization. By means of measuring the optical rotation of the crystallized acid of formula VI, one can obtain the configuration of this crystalline material. If this crystallized acid has a negative rotation, then this crystallized acid has the R configuration. After crystallization, each of the salts can be converted to the respective compounds of formula VI in the R and S configuration by hydrolysis with an acid. Among the preferred acids are dilute aqueous acids, i.e., from about 0.001N to 2N aqueous acids, such as aqueous sulfuric or aqueous hydrochloric acid. The configuration of formula VI which is produced by this method of resolution is carried out throughout the entire reaction scheme to produce the desired R of formula I. The separation of R and S isomers can also be achieved using an enzymatic ester hydrolysis of any lower alkyl esters corresponding to the compound of the formula VI (see, for example, Ahmar, M.; Girard, C.; Bloch, R., Tetrahedron Lett, 1989, 7053), which results in the formation of corresponding chiral acid and chiral ester. The ester and the acid can be separated by any conventional method of separating an acid from an ester. The preferred method of resolution of racemates of the compounds of the formula VI is via the formation of corresponding diastereomeric esters or amides. These diastereomeric esters or amides can be prepared by coupling the carboxylic acids of the formula VI with a chiral alcohol, or a chiral amine. This reaction can be carried out using any conventional method of coupling a carboxylic acid with an alcohol or an amine. The corresponding diastereomers of compounds of the formula VI can then be separated using any conventional separation methods. The resulting pure diastereomeric esters or amides can then be hydrolyzed to yield the corresponding pure R and S isomers. The hydrolysis reaction can be carried out using any conventional method to hydrolyze an ester or an amide without racemization.

All of the compounds described in the Examples activated glucokinase *in vitro* in accordance with the assay described in Example A.

On the basis of their capability of activating glucokinase, the compounds of above formula I can be used as medicaments for the treatment of type II diabetes. Therefore, as mentioned earlier, medicaments containing a compound of formula I are also an object of the present invention, as is a process for the manufacture of such medicaments, which process comprises bringing one or more compounds of formula I and, if desired, one or more other therapeutically valuable substances into a galenical administration form, e.g. by combining a compound of formula I with a pharmaceutically acceptable carrier and/or adjuvant.

The pharmaceutical compositions may be administered orally, for example in the form of tablets, coated tablets, dragées, hard or soft gelatine capsules, solutions, emulsions or suspensions. Administration can also be carried out rectally, for example using suppositories; locally or percutaneously, for example using ointments, creams, gels or solutions; or parenterally, e.g. intravenously, intramuscularly, subcutaneously, intrathecally or transdermally, using for example injectable solutions. Furthermore, administration can be carried out sublingually or as an aerosol, for example in the form of a spray. For the preparation of tablets, coated tablets, dragées or hard gelatine capsules the compounds of the present invention may be admixed with pharmaceutically inert, inorganic or organic excipients. Examples of suitable excipients for tablets, dragées or hard gelatine capsules include lactose, maize starch or derivatives thereof, talc or stearic acid or salts thereof. Suitable excipients for use with soft gelatine capsules include for example vegetable oils, waxes, fats, semi-solid or liquid polyols etc.; according to the nature of the active ingredients it may however be the case that no excipient is needed at all for soft gelatine capsules. For the preparation of solutions and syrups, excipients which may be used include for example water, polyols, saccharose, invert sugar and glucose. For injectable solutions, excipients which may be used include for example water, alcohols, polyols, glycerine, and vegetable oils. For suppositories, and local or percutaneous application, excipients which may be used include for example natural or hardened oils, waxes, fats and semi-solid or liquid polyols. The pharmaceutical compositions may also contain preserving agents, solubilising agents, stabilising agents, wetting agents, emulsifiers, sweeteners, colorants, odorants, salts for the variation of osmotic pressure, buffers, coating agents or antioxidants. As mentioned earlier, they may also contain other therapeutically valuable agents. It is a prerequisite that all adjuvants used in the manufacture of the preparations are non-toxic.

Preferred forms of use are intravenous, intramuscular or oral administration, most preferred is oral administration. The dosages in which the compounds of formula (I) are administered in effective amounts depend on the nature of the specific active ingredient, the age and the requirements of the patient and the mode of application. In general, dosages of about 1-100 mg/kg body weight per day come into consideration.

This invention will be better understood from the following examples, which are for purposes of illustration.

### Example 1

### {N-{4-[2-Cyclopentyl-1-(thiazol-2-ylcarbamoyl)-ethyl]-phenyl}-benzamide

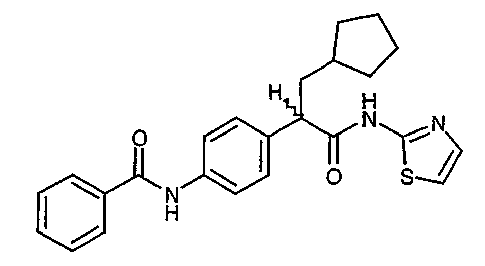

A solution of freshly prepared lithium diisopropylamide (430.55 mL of a 0.3M stock solution, 129.16 mmol) was cooled to -78°C and then treated with a solution of (4-nitrophenyl)-acetic acid ethyl ester (26.32 g, 125.83 mmol) in tetrahydrofuran/hexamethylphosphoramide (312.5 mL, 3:1). The resulting solution was stirred at -78°C for 45 min. At this time, the reaction was treated with a solution of iodomethylcyclopentane (27.75 g, 132.1 mmol) in hexamethylphosphoramide (27.75 mL). The mixture was stirred at -78°C for 4 h. The reaction was then warmed to 25°C and was stirred at 25°C for 16 h. At this time, the reaction mixture was quenched by the dropwise addition of a saturated aqueous ammonium chloride solution (250 mL). This mixture was concentrated *in vacuo,* diluted with water (250 mL), and extracted with ethyl acetate (3 x 300 mL). The combined organic extracts were washed with a saturated aqueous lithium chloride solution (2 x 250 mL), dried over magnesium sulfate, filtered, and concentrated *in vacuo.* Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as a yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (14.1 g,48.06 mmol) in tetrahydrofuran/water (300 mL, 3:1) was treated with lithium hydroxide (4.35 g, 103.67 mmol). The reaction was stirred at 25°C for 21 h. The tetrahydrofuran was then removed *in vacuo.* The residue was diluted with water (75 mL) and extracted with diethyl ether (3 x 75 mL). The aqueous layer was acidified to pH=1 with a 3N aqueous hydrochloric acid solution and then extracted with methylene chloride (3 x 75 mL). The combined organic extracts were washed with a saturated aqueous sodium chloride solution (2 x 100 mL), dried over magnesium sulfate, filtered, and concentrated *in vacuo* to give 3-cyclopentyl-2-(4-nitro-phenyl)-propionic acid (11.97 g, 93.6%) as a yellow solid: mp 119-125°C; EI-HRMS m/e calcd for C₁₄H₁₇NO₄ (M⁺) 263.1157, found 263.1162.

A solution of 3-cyclopentyl-2-(4-nitro-phenyl)-propionic acid (100 mg, 0.38 mmol) in ethyl acetate (50 mL) was treated with 10% palladium on activated carbon. The reaction mixture was stirred under 60 psi of hydrogen gas at 25°C for 16 h. The catalyst was then removed by filtration through a pad of celite and washed with ethyl acetate. The filtrate was concentrated *in vacuo* to afford 2-(4-amino-phenyl)-3-cyclopentyl-propionic acid (120 mg, 100%) as a white solid: mp 167-169°C; EI-HRMS m/e calcd for C₁₄H₁₉NO₂ (M⁺) 233.1415, found 233.1413.

A solution of 2-(4-amino-phenyl)-3-cyclopentyl-propionic acid (49 mg, 0.21 mmol) in tetrahydrofuran (5 mL) was treated with *N,N*-diisopropylethylamine (0.04 mL, 0.25 mmol) and benzoyl chloride (0.02 mL, 0.21 mmol). The reaction mixture was stirred at 25°C for 24 h. The reaction mixture was then concentrated *in vacuo.* Flash chromatography (Merck Silica gel 60, 230-400 mesh, 60/40 hexanes/ethyl acetate) afforded 2-(4-benzoylamino-phenyl)-3-cyclopentyl-propionic acid (41 mg, 57.9 %) as a white solid: mp 192.5-194°C; EI-HRMS m/e calcd for C₂₁H₂₃NO₃ (M⁺) 337.1677, found 337.1670.

A solution of 2-(4-benzoylamino-phenyl)-3-cyclopentyl-propionic acid (20.2 mg, 0.06 mmol), benzotriazol-1-yloxytris(dimethylamino)phosphonium hexafluorophosphate (39.8 mg, 0.09 mmol), and 2-aminothiazole (9.0 mg, 0.09 mmol) in methylene chloride (2 mL) at 25°C was treated with *N,N-*diisopropylethylamine (0.25 mL, 0.18 mmol). The reaction mixture was stirred at 25°C for 16 h. At this time, the mixture was poured into water (50 mL) and extracted with ethyl acetate (3 x 25 mL). The combined organic extracts were washed with a 1N aqueous hydrochloric acid solution (1 x 25 mL), dried over sodium sulfate, filtered, and concentrated *in vacuo.* Flash chromatography (Merck Silica gel 60, 230-400 mesh, 50/50 hexanes/ethyl acetate) afforded {N-{4-[2-cyclopentyl-1-(thiazol-2-ylcarbamoyl)-ethyl]-phenyl}-benzamide (95%) as a white solid: mp 285-290°C; EI-HRMS m/e calcd for C₂₄H₂₅N₃O₂S (M⁺) 419.1667, found 419.1667.

### Example 2

### 3-Cyclopentyl-N-thiazol-2-yl-2-[4-(2-thiophen-2-yl-acetylamino)-phenyl]-propionamide

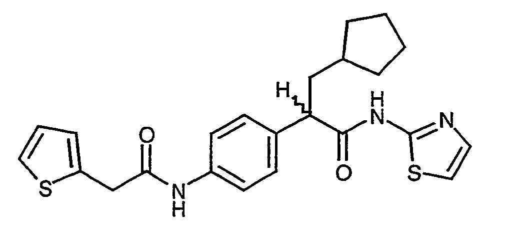

A solution of 2-(4-amino-phenyl)-3-cyclopentyl-propionic acid (prepared in Example 1, 49.0 mg, 0.21 mmol) in tetrahydrofuran (5 mL) at 25°C was treated with *N,N-*diisopropylethylamine (0.04 mL, 0.25 mmol) and thiophen-2-yl-acetyl chloride (0.02 mL, 0.21 mmol). The reaction was stirred at 25°C for 24 h. The reaction mixture was then concentrated *in vacuo.* Flash chromatography (Merck Silica gel 60, 230-400 mesh, 75/25 hexanes/ethyl acetate) afforded {N-{4-[2-cyclopentyl-1-(thiazol-2-ylcarbamoyl)-ethyl]-phenyl}-benzamide (61.0 mg, 81.2%) as a tan solid: mp 165-169°C; EI-HRMS m/e calcd for C₂₀H₂₃NO₃S (M⁺) 357.1399, found 357.1398.

A solution of 3-cyclopentyl-2-[4-(2-thiophen-2-yl-acetylamino)-phenyl]-propionic acid (76.6 mg, 0.21 mmol), benzotriazol-1-yloxytris(dimethylamino)phosphonium hexafluorophosphate (142.0 mg, 0.32 mmol) and 2-aminothiazole (32.1 mg, 0.32 mmol) in methylene chloride (1 mL) at 25°C was treated with triethylamine (0.9 mL, 0.64 mmol). The reaction mixture was stirred at 25°C for 16 h. This mixture was then poured into water (50 mL) and extracted with methylene chloride (2 x 25 mL). The combined organic extracts were washed with a 1N aqueous sodium hydroxide solution (1 x 25 mL), a 1N aqueous hydrochloric acid solution (1 x 25 mL), water (1 x 25 mL), and a saturated aqueous sodium chloride solution (3 x 25 mL). The organic layer was dried over sodium sulfate, filtered, and concentrated *in vacuo.* Flash chromatography (Merck Silica gel 60, 230-400 mesh, 60/40 hexanes/ethyl acetate) afforded 3-cyclopentyl-N-thiazol-2-yl-2-[4-(2-thiophen-2-yl-acetylamino)-phenyl]-propionamide (82.7 mg, 87.8%) as a tan solid: mp 220-221°C; EI-HRMS m/e calcd for C₂₃H₂₅N₃O₂S₂ (M⁺) 4391388, found 439.1379.

### Example 3

### 3-Cyclopentyl-2-[4-(4-nitro-benzenesulfonylamino)-phenyl]-N-thiazol-2-yl-propionamide

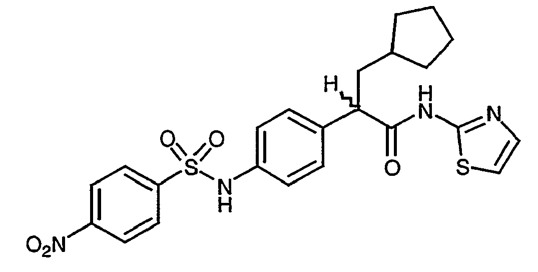

A solution of freshly prepared lithium diisopropylamide (430.55 mL of a 0.3M stock solution, 129.16 mmol) was cooled to -78°C and then treated with a solution of (4-nitrophenyl)-acetic acid ethyl ester (26.32 g, 125.83 mmol) in tetrahydrofuran/hexamethylphosphoramide (312.5 mL, 3:1). The resulting solution was stirred at -78°C for 45 min. At this time, the reaction mixture was treated with a solution of iodomethylcyclopentane (27.75 g, 132.1 mmol) in hexamethylphosphoramide (27.75 mL). The mixture was stirred at -78°C for 4 h. The reaction was then warmed to 25°C and was stirred at 25°C for 16 h. At this time, the reaction mixture was quenched by the dropwise addition of a saturated aqueous ammonium chloride solution (250 mL). This mixture was concentrated *in vacuo,* diluted with water (250 mL), and extracted with ethyl acetate (3 x 300 mL). The combined organic extracts were washed with a saturated aqueous lithium chloride solution (2 x 250 mL), dried over magnesium sulfate, filtered, and concentrated *in vacuo.* Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as a yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (14.1 g, 48.06 mmol) in tetrahydrofuran/water (300 mL, 3:1) was treated with lithium hydroxide (4.35 g, 103.67 mmol). The reaction was stirred at 25°C for 21 h. The tetrahydrofuran was then removed *in vacuo.* The residue was diluted with water (75 mL) and extracted with diethyl ether (3 x 75 mL). The aqueous layer was acidified to pH=1 with a 3N aqueous hydrochloric acid solution and was extracted with methylene chloride (3 x 75 mL). The combined organic extracts were washed with a saturated aqueous sodium chloride solution (2 x 100 mL), dried over magnesium sulfate, filtered, and concentrated *in vacuo* to give 3-cyclopentyl-2-(4-nitro-phenyl)-propionic acid (11.97 g, 93.6%) as a yellow solid: mp 119-125°C; EI-HRMS m/e calcd for C₁₄H₁₇NO₄ (M⁺) 263.1157, found 263.1162.

A solution of 3-cyclopentyl-2-(4-nitro-phenyl)-propionic acid (131 mg, 0.5 mmol) in methylene chloride (5.0 mL) was cooled to 0°C and then treated with a 2.0M solution of oxalyl chloride in methylene chloride (1 mL, 2.0 mmol) and a few drops of *N,N-*dimethylformamide. The reaction mixture was stirred at 0°C for 15 min and at 25°C for 30 min. The reaction mixture was then treated with a solution of 2-aminothiazole (110 mg, 1.0 mmol) in tetrahydrofuran (5 mL) and *N,N-*diisopropylethylamine (0.28 mL, 0.55 mmol). The solution was stirred at 25°C for 24 h. At this time, the reaction was concentrated *in vacuo.* Flash chromatography (Merck Silica gel 60, 230-400 mesh, 50/50 hexanes/ethyl acetate) afforded 3-cyclopentyl-2-(4-nitro-phenyl)-N-thiazol-2-yl-propionamide (38 mg, 22.4%) as a yellow solid: mp 186-187°C; EI-HRMS m/e calcd for C₁₇H₁₉N₃O₃S (M⁺) 345.1147, found 345.1148.

A solution of 3-cyclopentyl-2-(4-nitro-phenyl)-N-thiazol-2-yl-propionamide (345 mg, 1.0 mmol) in ethyl acetate (100 mL) was treated with 10% palladium on activated carbon (34.5 mg). The reaction mixture was stirred under 4,14 bar (60 psi) hydrogen gas at 25°C for 6 h. The catalyst was then removed by filtration through a pad of celite^{®} and was washed with ethyl acetate. The filtrate was concentrated *in vacuo* to give 2-(4-amino-phenyl)-3-cyclopentyl-N-thiazol-2-yl-propionamide (288.3 mg, 91.4%) as a yellow solid: mp 102-107°C; EI-HRMS m/e calcd for C₁₇H₂₁N₃OS (M⁺) 315.1405, found 315.1401. A solution of 2-(4-amino-phenyl)-3-cyclopentyl-N-thiazol-2-yl-propionamide (63.0 mg, 0.20 mmol) in tetrahydrofuran (10 mL) was treated with *N*,*N*-diisopropylethylamine (0.04 mL, 0.24 mmol) and 4-nitro-benzene sulfonyl chloride (49.0 mg, 0.20 mmol). The reaction mixture was stirred at 25°C for 21 h. At this time, the reaction was concentrated *in vacuo.* Flash chromatography (Merck Silica gel 60, 230-400 mesh, 50/50 hexanes/ethyl acetate) afforded 3-cyclopentyl-2-[4-(4-nitro-benzenesulfonylamino)-phenyl]-N-thiazol-2-yl-propionamide (47.5 mg, 47.5%) as a yellow solid: mp 120-125°C; FAB-HRMS m/e calcd for C₂₃H₂₄N₄O₅S₂ (M+H)⁺ 501.1266, found 501.1264.

### Example 4

### N-{4-[2-Cyclopentyl-1-(thiazol-2-ylcarbamoyl)-ethyl]-phenyl}-isonicotinamide

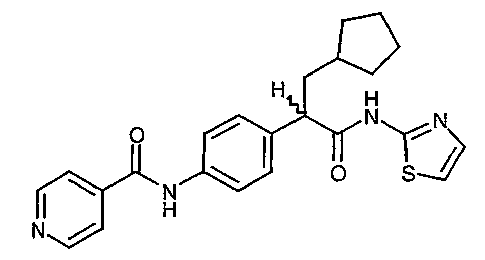

A solution of 2-(4-amino-phenyl)-3-cyclopentyl-N-thiazol-2-yl-propionamide (prepared in Example 3, 63.0 mg, 0.20 mmol) in tetrahydrofuran (10 mL) was treated with *N,N-*diisopropylethylamine (0.082 mL, 0.47 mmol) and isonicotinoyl chloride (35.6 mg, 0.20 mmol). The reaction mixture was stirred at 25°C for 24 h. At this time, the reaction was concentrated *in vacuo.* Flash chromatography (Merck Silica gel 60, 230-400 mesh, 90/10 ethyl acetate/methanol) afforded N-{4-[2-cyclopentyl-1-(thiazol-2-ylcarbamoyl)-ethyl]-phenyl}-isonicotinamide (65.5 mg, 77.9%) as a white solid: mp 225-230°C; FAB-HRMS m/e calcd for C₂₃H₂₄N₄O₂S (M+H)⁺ 421.1698, found 421.1698.

### Example 5

### N-{4-[2-Cyclopentyl-1-(thiazol-2-ylcarbamoyl)-ethyl]-phenyl}-4-nitro-benzamide

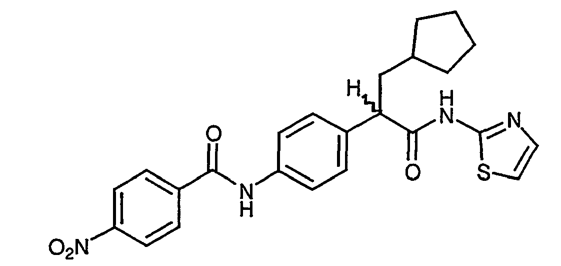

A solution of 2-(4-amino-phenyl)-3-cyclopentyl-N-thiazol-2-yl-propionamide (prepared in Example 3, 63.0 mg, 0.20 mmol) in tetrahydrofuran (10 mL) was treated with *N,N-*diisopropylethylamine (0.04 mL, 0.24 mmol) and 4-nitro-benzoyl chloride (49.0 mg, 0.26 mmol). The reaction mixture was stirred at 25°C for 21 h. At this time, the reaction was concentrated *in vacuo.* The residue was triturated with diethyl ether to afford N-{4-[2-cyclopentyl-1-(thiazol-2-ylcarbamoyl)-ethyl]-phenyl}-4-nitro-benzamide (73.7 mg, 79.3%) as a pale yellow solid: mp 236-238°C; FAB-HRMS m/e calcd for C₂₄H₂₄N₄O₄S (M+H)⁺ 465.1596, found 465.1617.

### Example 6

### N-{4-[2-Cyclopentyl-1-(thiazol-2-ylcarbamoyl)-ethyl]-phenyl}-oxalamic acid methyl ester

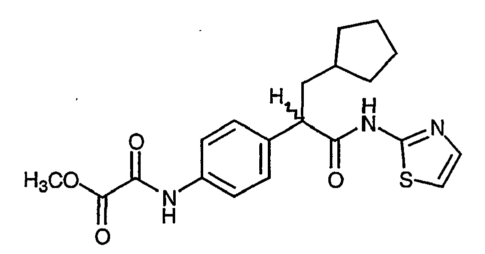

A solution of 2-(4-amino-phenyl)-3-cyclopentyl-N-thiazol-2-yl-propionamide (prepared in Example 3, 78 mg, 0.25 mmol) in tetrahydrofuran (10 mL) was treated with *N,N-*diisopropylethylamine (0.05 mL, 0.30 mmol) and methyl oxalyl chloride (0.02 mL, 0.25 mmol). The reaction mixture was stirred at 25°C for 16 h. At this time, the reaction was concentrated *in vacuo.* High pressure liquid chromatography (Chromegasphere SI-60, 10µM, 60Å, 25cm x 23cm ID, 70/30 heptane/ethyl acetate) afforded N-{4-[2-cyclopentyl-1-(thiazol-2-ylcarbamoyl)-ethyl]-phenyl}-oxalamic acid methyl ester (13.7 mg, 13.6%) as a white solid: mp 95-98°C; EI-HRMS m/e calcd for C₂₀H₂₃N₃O₄S (M⁺) 401.1409, found 401.1402.

### Example 7

### Acetic acid {4-[2-cyclopentyl-1-(thiazol-2-ylcarbamoyl)-ethyl]-phenylcarbamoyl}-methyl ester

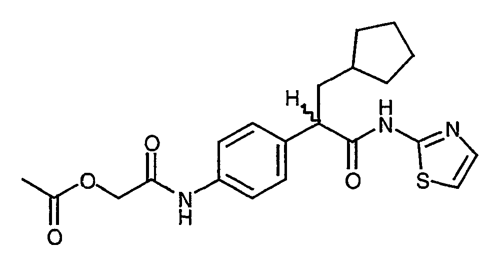

A solution of 2-(4-amino-phenyl)-3-cyclopentyl-N-thiazol-2-yl-propionamide (prepared in Example 3, 105 mg, 0.33 mmol) in tetrahydrofuran (10 mL) was treated with *N,N-*diisopropylethylamine (0.068 mL, 0.40 mmol) and acetoxy acetyl chloride (0.03 mL, 0.33 mmol). The reaction mixture was stirred at 25°C for 5h. At this time, the reaction was concentrated *in vacuo.* High pressure liquid chromatography (Chromegasphere SI-60, 10µM, 60Å, 25cm x 23cm ID, 50/50 heptane/ethyl acetate) afforded acetic acid {4-[2-cyclopentyl-1-(thiazol-2-ylcarbamoyl)-ethyl]-phenylcarbamoyl}-methyl ester (31.4 mg, 22.7%) as a white solid: mp 90-95°C; EI-HRMS m/e calcd for C₂₁H₂₅N₃O₄S (M⁺) 415.1565, found 415.1567.

### Example 8

### N-{4-[2-Cyclopentyl-1-(thiazol-2-ylcarbamoyl)-ethyl]-phenyl}-dimethylsulfamide

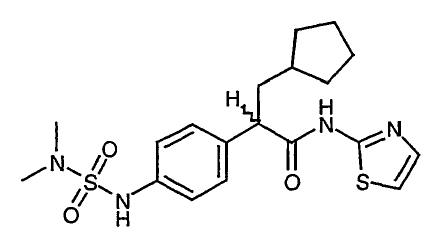

A solution of 2-(4-amino-phenyl)-3-cyclopentyl-N-thiazol-2-yl-propionamide (prepared in Example 3, 105 mg, 0.33 mmol) in pyridine (5 mL) was treated with dimethylsulfamoyl chloride (0.04 mL, 0.38 mmol). The reaction mixture was stirred at 25°C for 24 h. At this time, the reaction was concentrated *in vacuo.* The residue was dissolved in methylene chloride, and the organic phase was washed with a 1N aqueous hydrochloric acid solution, a saturated aqueous sodium bicarbonate solution, and a saturated aqueous sodium chloride solution. The organic layer was dried over magnesium sulfate, filtered, and concentrated *in vacuo.* High pressure liquid chromatography (Chromegasphere SI-60, 10µM, 60Å, 25cm x 23cm ID, 60/40 heptane/ethyl acetate) afforded the N-{4-[2-cyclopentyl-1-(thiazol-2-ylcarbamoyl)-ethyl]-phenyl}-dimethylsulfamide (21.3%) as a white solid: mp 110-112°C; FAB-HRMS m/e calcd for C₁₉H₂₆N₄O₃S₂ (M+H)⁺ 423.1524, found 423.1524.

### Example 9

### N-{4-[2-Cyclopentyl-1-(thiazol-2-ylcarbamoyl)-ethyl]-phenyl}-nicotinamide

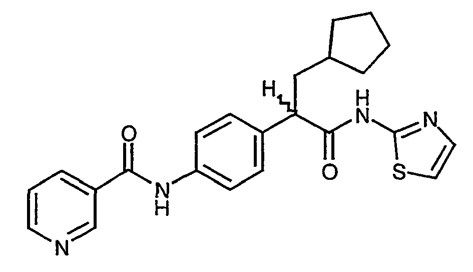

A solution of 2-(4-amino-phenyl)-3-cyclopentyl-N-thiazol-2-yl-propionamide (prepared in Example 3, 63.0 mg, 0.20 mmol) in tetrahydrofuran (10 mL) was treated with *N,N-*diisopropylethylamine (0.082 mL, 0.48 mmol) and nicotinoyl chloride hydrochloride (35.6 mg, 0.20 mmol). The reaction mixture was stirred at 25°C for 24 h. At this time, the reaction was concentrated *in vacuo.* Flash chromatography (Merck Silica gel 60, 230-400 mesh, 90/10 ethyl acetate/methanol) afforded N-{4-[2-cyclopentyl-1-(thiazol-2-ylcarbamoyl)-ethyl]-phenyl}-nicotinamide (58.9 mg, 70%) as a white solid: mp 240-242°C; EI-HRMS m/e calcd for C₂₃H₂₄N₄O₂S (M⁺) 420.1619, found 420.1625.

### Example 10

### 2-(3-Cyclopentyl-2-{4-[(pyridine-3-carbonyl)-amino]-phenyl} propionylamino)-thiazole-4-carboxylic acid ethyl ester

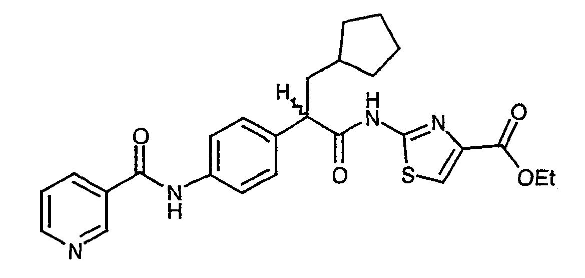

A solution of freshly prepared lithium diisopropylamide (430.55 mL of a 0.3M stock solution, 129.16 mmol) was cooled to -78°C and then treated with a solution of (4-nitrophenyl)-acetic acid ethyl ester (26.32 g, 125.83 mmol) in tetrahydrofuran/hexamethylphosphoramide (312.5 mL, 3:1). The resulting solution was stirred at -78°C for 45 min. At this time, the reaction was treated with a solution of iodomethylcyclopentane (27.75 g, 132.1 mmol) in hexamethylphosphoramide (27.75 mL). The mixture was stirred at -78°C for 4 h. The reaction was then warmed to 25°C and was stirred at 25°C for 16 h. At this time, the reaction mixture was quenched by the dropwise addition of a saturated aqueous ammonium chloride solution (250 mL). This mixture was concentrated *in vacuo,* diluted with water (250 mL), and extracted with ethyl acetate (3 x 300 mL). The combined organic extracts were washed with a saturated aqueous lithium chloride solution (2 x 250 mL), dried over magnesium sulfate, filtered, and concentrated *in vacuo.* Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as an yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (14.1 g,48.06 mmol) in tetrahydrofuran/water (300 mL, 3:1) was treated with lithium hydroxide (4.35 g, 103.67 mmol). The reaction was stirred at 25°C for 21 h. The tetrahydrofuran was then removed *in vacuo.* The residue was diluted with water (75 mL) and extracted with ether (3 x 75 mL). The aqueous layer was acidified to pH=1 with a 3N aqueous hydrochloric acid solution and was extracted with methylene chloride (3 x 75 mL). The combined organic extracts were washed with a saturated aqueous sodium chloride solution (2 x 100 mL), dried over magnesium sulfate, filtered, and concentrated *in vacuo* to afford 3-cyclopentyl-2-(4-nitro-phenyl)-propionic acid (11.97 g, 93.6%) as a yellow solid: mp 119-125°C; EI-HRMS m/e calcd for C₁₄H₁₇NO₄ (M⁺) 263.1157, found 263.1162.

A solution of 3-cyclopentyl-2-(4-nitro-phenyl)-propionic acid (100 mg, 0.38 mmol) in ethyl acetate (50 mL) was treated with 10% palladium on activated carbon. The reaction mixture was stirred unde 4,14 bar (60 psi) of hydrogen gas at 25°C for 16 h. The catalyst was removed by filtration through a pad of celite^{®} and was washed with ethyl acetate. The filtrate was concentrated *in vacuo* to afford 2-(4-amino-phenyl)-3-cyclopentyl-propionic acid (120 mg, 100%) as a white solid: mp 167-169°C; EI-HRMS m/e calcd for C₁₄H₁₉NO₂ (M⁺) 233.1415, found 233.1413.

A solution of 2-(4-amino-phenyl)-3-cyclopentyl-propionic acid (130 mg, 0.56 mmol) in tetrahydrofuran (10 mL) at 25°C was treated with *N,N-*diisopropylethylamine (0.23 mL, 1.34 mmol) and nicotinoyl chloride hydrochloride (99 mg, 0.54 mmol). The reaction mixture was stirred at 25°C for 48 h. At this time, the reaction was concentrated *in vacuo.* High pressure liquid chromatography (Chromegasphere SI-60, 10µM, 60Å, 25cm x 23cm ID, 90/10 ethyl acetate/heptane) afforded 3-cyclopentyl-2-{4-[(pyridine-3-carbonyl)-amino]-phenyl}-propionic acid (40.9 mg, 21.7%) as a yellow solid: mp 160-163°C; EI-HRMS m/e calcd for C₂₁H₂₃NO₃ (M⁺) 337.1677, found 337.1670.

A solution of 3-cyclopentyl-2-{4-[(pyridine-3-carbonyl)-amino]-phenyl}-propionic acid (233 mg, 0.66 mmol) in methylene chloride (10 mL) was cooled to 0°C and then treated with a 2.0M solution of oxalyl chloride in methylene chloride (0.36 mL, 0.72 mmol) and a few drops of *N,N*-dimethylformamide. The reaction mixture was stirred at 0°C for 15 min and at 25°C for 30 min. The reaction mixture was then treated with a solution of 2-amino-thiazole-4-carboxylic acid ethyl ester (367 mg, 1.45 mmol) in tetrahydrofuran (5 mL) and *N,N*-diisopropylethylamine (0.40 mL, 2.31 mmol). This solution was stirred at 25°C for 48 h. At this time, the reaction was concentrated *in vacuo.* High pressure liquid chromatography (Chromegasphere SI-60, 10µM, 60Å, 25cm x 23cm ID, 95/5 ethyl acetate/heptane) afforded 2-(3-cyclopentyl-2-{4-[(pyridine-3-carbonyl)-amino]-phenyl} propionylamino)-thiazole-4-carboxylic acid ethyl ester (40.5 mg, 12.5%) as an off-white: mp 222-223°C; EI-HRMS m/e calcd for C₂₆H₂₈N₄O₄S (M⁺) 492.1831, found 492.1835.

### Example 11

### [2-(3-Cyclopentyl-2-{4-[(pyridine-3-carbonyl)-amino]-phenyl}-propionylamino)-thiazol-4-yl]-acetic acid ethyl ester

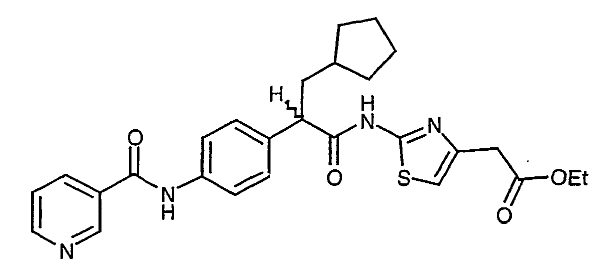

A solution of 3-cyclopentyl-2-{4-[(pyridine-3-carbonyl)-amino]-phenyl}-propionic acid (prepared in Example 10, 169 mg, 0.50 mmol) in methylene chloride (10 mL) at 25°C was treated with triethylamine (0.21 mL, I.3 mmol), benzotriazol-1-yloxytris(dimethylamino)phosphonium hexafluorophosphate (332 mg, 0.75 mmol), and (2-amino-thiazol-4-yl)-acetic acid ethyl ester (140 mg, 0.75 mmol). The reaction mixture was stirred at 25°C for 20 h. At this time, the reaction was diluted with methylene chloride (50 mL). This solution was washed with a 1N aqueous hydrochloric acid solution (1 x 15 mL), water (1 x 15 mL), and a saturated aqueous sodium chloride solution (2 x 25 mL). The organic layer was dried over magnesium sulfate, filtered, and concentrated *in vacuo.* Flash chromatography (Merck Silica gel 60, 230-400 mesh, 100% ethyl acetate) afforded [2-(3-cyclopentyl-2-{4-[(pyridine-3-carbonyl)-amino]-phenyl}-propionylamino)-thiazol-4-yl]-acetic acid ethyl ester (100.1 mg, 39.5%) as white solid: mp 195-200°C; EI-HRMS m/e calcd for C₂₇H₃₀N₄O₄S (M⁺) 506.1987, found 506.1985.

### Example 12

### 3-Cyclopentyl-2-(4-methanesulfonylamino-phenyl)-N-thiazol-2-yl-propionamide

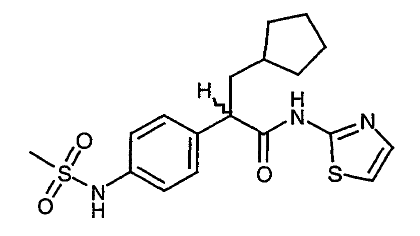

A solution of 2-(4-amino-phenyl)-3-cyclopentyl-N-thiazol-2-yl-propionamide (prepared in Example 3, 158 mg, 0.5 mmol) in pyridine (5 mL) at 25°C was treated with methanesulfonyl chloride (50 µL, 0.57 mmol). The reaction was stirred at 25°C for 7 h and was then concentrated *in vacuo.* High pressure liquid chromatography (Chromegasphere SI-60, 10µM, 60Å, 25cm x 23cm ID, 40/60 heptane/ethyl acetate) afforded 3-cyclopentyl-2-(4-methanesulfonylamino-phenyl)-N-thiazol-2-yl-propionamide (98.2 mg, 49.9%) as a tan solid: mp 85-90°C; EI-HRMS m/e calcd for C₁₈H₂₃N₃O₃S (M⁺) 393.1180, found 393.1185.

### Example 13

### 3-Cyclopentyl-N-thiazol-2-yl-2-(4-trifluoromethanesulfonylamino-phenyl)-propionamide

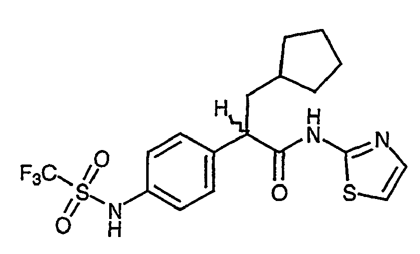

A solution of 2-(4-amino-phenyl)-3-cyclopentyl-N-thiazol-2-yl-propionamide (prepared in Example 3, 158 mg, 0.5 mmol) in pyridine (5 mL) at 25°C was treated with trifluoromethanesulfonyl chloride (60 µL, 0.57 mmol). The reaction was stirred at 25°C for 7 h and was then concentrated *in vacuo.* High pressure liquid chromatography (Chromegasphere SI-60, 10µM, 60Å, 25cm x 23cm ID, 40/60 heptane/ethyl acetate) afforded the 3-cyclopentyl-N-thiazol-2-yl-2-(4-trifluoromethanesulfonylamino-phenyl)-propionamide (93.8 mg, 41.9%) as a tan solid: mp 70-74°C; EI-HRMS m/e calcd for C₁₈H₂₀F₃N₃O₃S₂ (M⁺) 447.0898, found 447.0894.

### Example 14

### 3-Cyclopentyl-N-thiazol-2-yl-2-[4-(2,2,2-trifluoro-ethanesulfonylamino)-phenyl]-propionamide

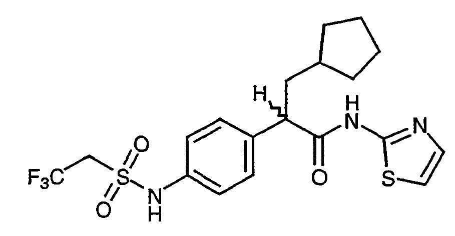

A solution of 2-(4-amino-phenyl)-3-cyclopentyl-N-thiazol-2-yl-propionamide (prepared in Example 3, 158 mg, 0.5 mmol) in pyridine (5 mL) at 25°C was treated with 2,2,2-trifluoroethanesulfonyl chloride (63.5 µL, 0.57 mmol). The reaction was stirred at 25°C for 48 h and was then concentrated *in vacuo.* High pressure liquid chromatography (Chromegasphere SI-60, 10µM, 60Å, 25cm x 23cm ID, 40/60 heptane/ethyl acetate) afforded the 3-cyclopentyl-N-thiazol-2-yl-2-[4-(2,2,2-trifluoro-ethanesulfonylamino)-phenyl]-propionamide (98.1 mg, 42.4%) as a light yellow oil: EI-HRMS m/e calcd for C₁₉H₂₂F₃N₃O₃S₂ (M⁺) 461.1054, found 461.1064.

### Example 15

### N-{4-[2-Cyclopentyl-1-(pyridin-2-ylcarbamoyl)-ethyl]-phenyl}-nicotinamide

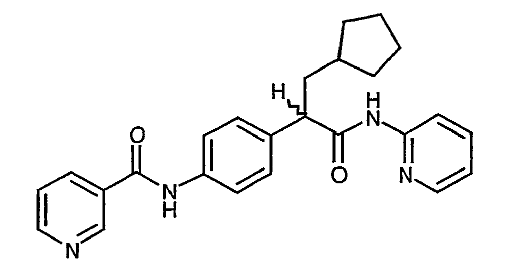

A solution of 3-cyclopentyl-2-(4-nitro-phenyl)-propionic acid (prepared in Example 1, 263 mg, 1.0 mmol) in methylene chloride (5 mL) was cooled to 0°C and then treated with a 2.0M solution of oxalyl chloride in methylene chloride (0.60 mL, 1.2 mmol) and a few drops of *N*,*N*-dimethylformamide. The reaction mixture was stirred at 0°C for 15 min and at 25°C for 1 h. The reaction was then treated with a solution of 2-aminopyridine (207 mg, 2.2 mmol) in tetrahydrofuran (5 mL) and *N,N*-diisopropylethylamine (0.42 mL, 2.5 mmol). This solution was stirred at 25°C for 24 h. At this time, the reaction was concentrated *in vacuo.* Flash chromatography (Merck Silica gel 60, 230-400 mesh, 80/20 hexanes/ethyl acetate) afforded 3-cyclopentyl-2-(4-nitro-phenyl)-N-pyridin-2-yl-propionamide (110.2 mg, 32.5%) as a white solid: mp 152-154°C; EI-HRMS m/e calcd for C₁₉H₂₁N₃O₃ (M⁺) 339.1582, found 339.1581.

A solution of 3-cyclopentyl-2-(4-nitro-phenyl)-N-pyridin-2-yI-propionamide (130 mg, 0.38 mmol) in ethyl acetate (50 mL) and methanol (5 mL) was treated with 10% palladium on activated carbon (50 mg). The reaction mixture was shaken unden 4,14 bar (60 psi) of hydrogen gas at 25°C for 18 h. The catalyst was then removed by filtration through a pad of celite^{®} and washed with ethyl acetate. The filtrate was concentrated *in vacuo* to afford 2-(4-amino-phenyl)-3-cyclopentyl-N-pyridin-2-yl-propionamide (99.9 mg, 84.3%) as a tan oil: EI-HRMS m/e calcd for C₂₁H₂₃N₃O (M⁺) 309.1841, found 309.1849.

A solution of 2-(4-amino-phenyl)-3-cyclopentyl-N-pyridin-2-yl-propionamide (81.7 mg, 0.26 mmol) in tetrahydrofuran (10 mL) at 25°C was treated with *N,N-*diisopropylethylamine (0.11 mL, 0.63 mmol) and nicotinoyl chloride hydrochloride (47 mg, 0.26 mmol). The resulting reaction mixture was stirred at 25°C for 48 h. At this time, the reaction was concentrated *in vacuo.* High pressure liquid chromatography (Chromegasphere SI-60, 10µM, 60Å, 25cm x 23cm ID, 90/10 ethyl acetate/heptane) afforded N-{4-[2-cyclopentyl-1-(pyridin-2-ylcarbamoyl)-ethyl]-phenyl}-nicotinamide (40.9 mg, 21.7%) as a yellow solid: mp 160-163°C; EI-HRMS m/e calcd for C₂₅H₂₆N₄O₂ (M⁺) 414.2055, found 414.2056.

### Example 16

### 6-(3-Cyclopentyl-2-{4-[(pyridine-3-carbonyl)-amino]-phenyl}-propionylamino)-nicotinic acid methyl ester

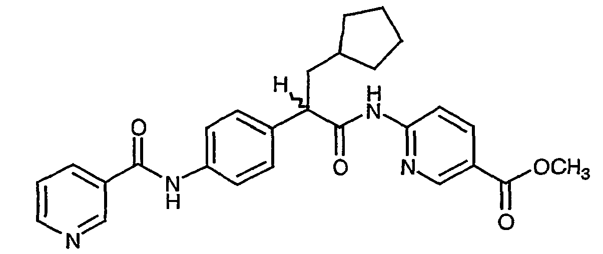

A solution of 3-cyclopentyl-2-(4-nitro-phenyl)-propionic acid (prepared in Example 1, 526 mg, 2.0 mmol) in methylene chloride (20 mL) was cooled to 0°C and then treated with a 2.0M solution of oxalyl chloride in methylene chloride (1.2 mL, 2.4 mmol) and a few drops of *N,N-*dimethylformamide. The reaction mixture was stirred at 0°C for 10 min and at 25°C for 30 min. The reaction mixture was then treated with a solution of 6-amino-nicotinic acid methyl ester (532 mg, 3.5 mmol) and *N,N*-diisopropylethylamine (0.84 mL, 4.82 mmol) in tetrahydrofuran (10 mL). The reaction mixture was stirred at 25°C for 48 h. At this time, the reaction was concentrated *in vacuo.* High pressure liquid chromatography (Chromegasphere SI-60, 10µM, 60Å, 25cm x 23cm ID, 50/50 heptane/ethyl acetate) afforded 6-[3-cyclopentyl-2-(4-nitro-phenyl)-propionylamino]-nicotinic acid methyl ester (353.9 mg, 44.6%) as a pale orange oil: EI-HRMS m/e calcd for C₂₁H₂₃N₃O₅ (M⁺) 397.1637, found 397.1631.

A solution of 6-[3-cyclopentyl-2-(4-nitro-phenyl)-propionylamino]-nicotinic acid methyl ester (300 mg, 0.75 mmol) in ethyl acetate (30 mL) and methanol (5 mL) was treated with 10% palladium on activated carbon (30 mg). The reaction mixture was shaken under 4,14 bar (60 psi) of hydrogen gas at 25°C for 16 h. The catalyst was then removed by filtration through a pad of celite^{®} and washed with ethyl acetate. The filtrate was concentrated *in vacuo* to afford 6-[2-(4-amino-phenyl)-3-cyclopentyl-propionylamino]-nicotinic acid methyl ester (277.4 mg, quant) as a pale yellow glass: mp 65-68°C; EI-HRMS m/e calcd for C₂₁H₂₅N₃O₃ (M⁺) 367.1893, found 367.1899.

A solution of 6-[2-(4-amino-phenyl)-3-cyclopentyl-propionylamino]-nicotinic acid methyl ester (236.1mg, 0.65 mmol) in tetrahydrofuran (15 mL) at 25°C was treated with *N,N*-diisopropylethylamine (0.27 mL, 1.54 mmol) and nicotinoyl chloride hydrochloride (115 mg, 0.64 mmol). The reaction mixture was stirred at 25°C for 48 h. At this time, the reaction was concentrated *in vacuo.* Flash chromatography (Merck Silica gel 60, 230-400 mesh, 90/10 ethyl acetate/hexanes) afforded 6-(3-cyclopentyl-2-{4-[(pyridine-3-carbonyl)-amino]-phenyl}-propionylamino)-nicotinic acid methyl ester (219.6 mg, 72.3%) as a white solid: mp 110-115°C; EI-HRMS m/e calcd for C₂₇H₂₈N₄O₄ (M⁺) 472.2110, found 472.2109.

### Example 17

### 6-(3-Cyclopentyl-2-{4-[(pyridine-3-carbonyl)-amino]-phenyl}-propionylamino)-nicotinic acid

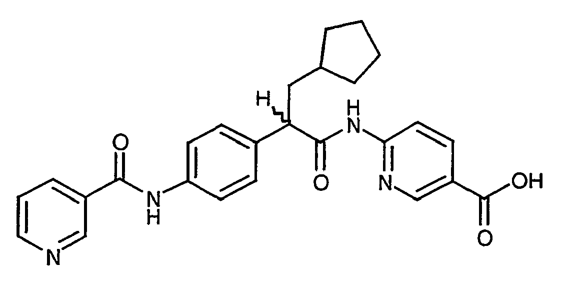

A solution of 6-(3-cyclopentyl-2-{4-[(pyridine-3-carbonyl)-amino]-phenyl}-propionylamino)-nicotinic acid methyl ester (prepared in Example 16, 87.2 mg, 0.18 mmol) in tetrahydrofuran (8 mL) and water (2 mL) was treated with lithium hydroxide (17.0 mg, 0.41 mmol). The reaction was stirred at 25°C for 20 h. At this time, the reaction was concentrated *in vacuo.* The residue was diluted with water (25 mL) and extracted with diethyl ether (1 x 20 mL). The aqueous layer was acidified to pH=1 with a 3N aqueous hydrochloric acid solution and was extracted with methylene chloride (3 x 50 mL). The combined organic extracts were washed with water (1 x 50 mL) and a saturated aqueous sodium chloride solution (2 x 50 mL), dried over magnesium sulfate, filtered, and concentrated *in vacuo.* High pressure liquid chromatography (Chromegasphere SI-60, 10µM, 60Å, 25cm x 23cm ID, 100% ethyl acetate with acetic acid) afforded 6-[3-cyclopentyl-2-(4-nitro-phenyl)-propionylamino]-nicotinic acid (6.4 mg, 7.5%) as a pale yellow oil: EI-HRMS m/e calcd for C₁₆H₂₆N₄O₄ (M⁺) 458.1954, found 458.1967.

### Biological Activity Example: In Vitro Glucokinase Activity

*Glucokinase Assay:* Glucokinase (GK) was assayed by coupling the production of glucose-6-phosphate to the generation of NADH with glucose-6-phosphate dehydrogenase (G6PDH, 0.75-1 kunits/mg; Boehringer Mannheim, Indianapolis, IN) from *Leuconostoc mesenteroides* as the coupling enzyme (Scheme 2). Recombinant 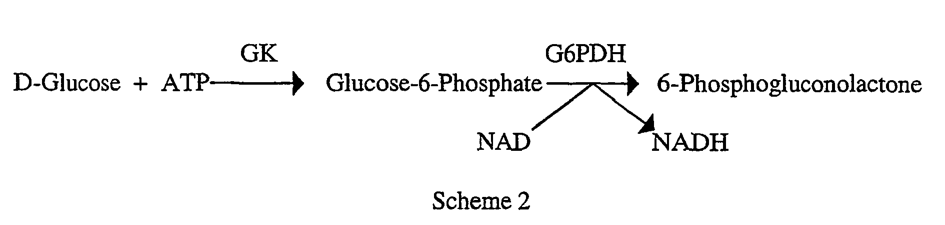 Human liver GK1 was expressed in *E. coli* as a glutathione S-transferase fusion protein (GST-GK) [Liang et al, 1995] and was purified by chromatography over a glutathione-Sepharose 4B affinity column using the procedure provided by the manufacturer (Amersham Pharmacia Biotech, Piscataway, NJ). Previous studies have demonstrated that the enzymatic properties of native GK and GST-GK are essentially identical (Liang et al, 1995; Neet et al., 1990).

The assay was conducted at 25° C in a flat bottom 96-well tissue culture plate from Costar (Cambridge, MA) with a final incubation volume of 120 µl. The incubation mixture contained: 25 mM Hepes buffer (pH, 7.1), 25 mM KCl, 5 mM D-glucose, 1mM ATP, 1.8 mM NAD, 2 mM MgCl₂, 1 µM sorbitol-6-phosphate, 1 mM dithiothreitol, test drug or 10% DMSO, 1.8 unit/ml G6PDH, and GK (see below). All organic reagents were >98 % pure and were from Boehringer Mannheim with the exceptions of D-glucose and Hepes that were from Sigma Chemical Co, St Louis, MO. Test compounds were dissolved in DMSO and were added to the incubation mixture minus GST-GK in a volume of 12 µl to yield a final DMSO concentration of 10%. This mix was preincubated in the temperature controlled chamber of a SPECTRAmax 250 microplate spectrophotometer (Molecular Devices Corporation, Sunnyvale, CA) for 10 minutes to allow temperature equilibrium and then the reaction was started by the addition of 20 µl GST-GK.

After addition of enzyme, the increase in optical density (OD) at 340 nm was monitored over a 10 minute incubation period as a measure of GK activity. Sufficient GST-GK was added to produce an increase in OD₃₄₀ of 0.08 to 0.1 units over the 10 minute incubation period in wells containing 10% DMSO, but no test compound. Preliminary experiments established that the GK reaction was linear over this period of time even in the presence of activators that produced a 5-fold increase in GK activity. The GK activity in control wells was compared with the activity in wells containing test GK activators, and the concentration of activator that produced a 50% increase in the activity of GK, i.e., the SC_{1.5}, was calculated. All of the compounds of formula I described in the Synthesis Examples had an SC_{1.5} less than or equal to 30 µM.

### Example A

Tablets containing the following ingredients can be produced in a conventional manner:

| Ingredients | mg per tablet |
|---|---|
| Compound of formula (I) | 10.0-100.0 |
| Lactose | 125.0 |
| Corn starch | 75.0 |
| Talc | 4.0 |
| Magnesium stearate | 1.0 |

### Example B

Capsules containing the following ingredients can be produced in a conventional manner:

| Ingredients | mg per capsule |
|---|---|
| Compound of formula (I) | 25.0 |
| Lactose | 150.0 |
| Com starch | 20.0 |
| Talc | 5.0 |

## Claims

1. An amide selected from the group consisting of a compound of the formula 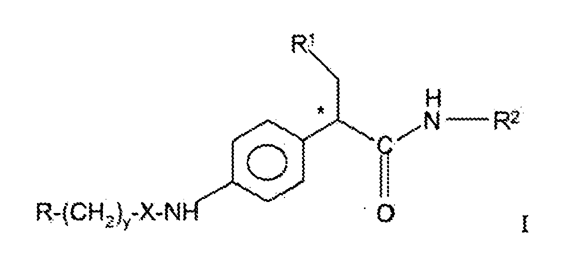 wherein
X is 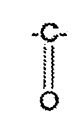 or 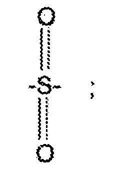
R is perfluoro-lower alkyl, lower alkyl, 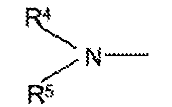
lower alkoxycarbonyl, a heteroaromatic ring, connected by a ring carbon atom, containing from 5 to 6 ring members with from 1 to 3 heteroatoms selected from the group consisting of oxygen, sulfur and nitrogen, unsubstituted aryl containing 6 or 10 ring carbon atoms, a nitro or a lower alkyl substituted aryl which aryl contains 6 or 10 ring carbon atoms or a saturated 5- or 6-membered cycloheteroalkyl ring, connected by a ring carbon atom, containing from 1 to 2 heteroatoms selected from the group consisting of oxygen, nitrogen, sulfur or cycloalkyl having 5 or 6 carbon atoms;
R¹ is cycloalkyl having 5 or 6 carbon atoms;
R² is a five- or six-membered heteroaromatic ring connected by a ring carbon atom to the amide group to the remainder of the compound, which heteroaromatic ring contains from 1 to 3 heteroatoms selected from the group consisting of oxygen, sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent selected from lower alkyl and 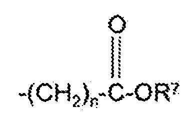 n and y are independently an integer from 0 to 4;
R⁴, R⁵ and R⁷ are, independently hydrogen or lower alkyl; and
* denotes the asymmetric carbon atom;
and wherein "lower" means 1 to 7 carbon atoms;
and pharmaceutically acceptable salt thereof.

2. The amide of claim 1 wherein said compound is 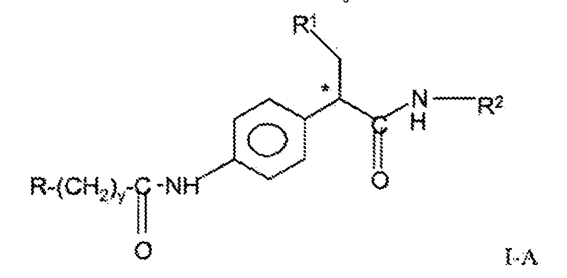 wherein R. R¹, R² and y are as defined in claim 1.

3. The amide of claim 1 wherein said compound is 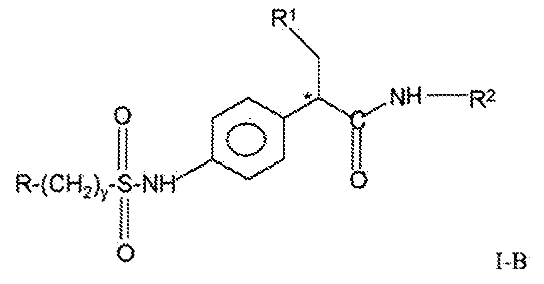 wherein R, R¹, R² and y are as defined in claim 1.

4. The amide of any of claims 1 to 3 wherein R¹ is cyclopentyl.

5. The amide of any of claims 1 to 4 wherein R² is a five-membered heteroaromatic ring connected by a ring carbon atom to the amide group to the remainder of the compound, which heteroaromatic ring contains from 1 to 3 heteroatoms selected from the group consisting of oxygen, sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent selected from lower alkyl and (CH₂)ₙ-C(O)OR⁷; and "lower", n and R⁷ are as defined in claim 1.

6. The amide of any of claims 1 to 4 wherein R² is a six-membered heteroaromatic ring connected by a ring carbon atom to the amide group to the remainder of the compound, which heteroaromatic ring contains from 1 to 3 heteroatoms selected from the group consisting of oxygen, sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent selected from tower alkyl and -(CH₂)ₙ-C(O)OR⁷; and "lower", n and R⁷ are as defined in claim 1.

7. The amide of any of claims 1 to 4 wherein R² is thiazolyl connected by a ring carbon atom to the amide group to the remainder of the compound, said thiazolyl being optionally monosubstituted at a position on a ring carbon atom other than adjacent to said connecting carbon atom with a substituent selected from lower alkyl or -(CH₂)ₙ-C(O)OR⁷; and "lower", n and R⁷ are as defined in claim 1.

8. The amide of any of claims 1 to 4 wherein R² is pyridyl connected by a ring carbon atom to the amide group to the remainder of the compound, said pyridyl being optionally monosubstituted at a position on a ring carbon atom other than adjacent to said connecting carbon atom with a substituent selected from lower alkyl or -(CH₂)ₙ-C(O)OR⁷, and "lower", n and R⁷ are as defined in claim 1.

9. The amide of any of claims 1 to 8 wherein R is phenyl, naphthyl or nitro substituted phenyl or naphthyl.

10. The amide of any of claims 1 to 8 wherein R is nitro substituted phenyl.

11. The amide of any of claims 1 to 8 wherein R. is unsubstituted phenyl.

12. The amide of any of claims 1 to 8 wherein R is a heteroaromatic ring, connected by a ring carbon atom, containing from 5 to 6 ring members with from 1 to 3 heteroatoms selected from the group consisting of oxygen, sulfur and nitrogen.

13. The amide of any of claims 1. to 8 wherein R. is thienyl or pyridyl.

14. The amide of any of claims 1 to 8 wherein R is lower alkoxy carbonyl, and "lower" means 1 to 7 carbon atoms.

15. The amide of any of claims 1 to 8 wherein R is lower alkyl or perfluoro lower alkyl, and "lower" means 1 to 7 carbon atoms.

16. The amide of any of claims I to 8 wherein R is -N(R⁴,R⁵) and R⁴ and R⁵ are as defined in claim 1.

17. The amide of any of claims 1 to 16 wherein n and y are independently 0 or 1.

18. The amide of any claims 1 to 17 wherein R⁴ and R⁵ are lower alkyl, and "lower" means 1 to 7 carbon atoms.

19. The amide of any of claims to 17, wherein X is -C(O)- or S(O)₂-; R is perfluoro-lower alkyl, lower alkyl, -N(R⁴,R⁵), lower alkoxycarbonyl, a heteroaromatic ring, connected by a ring carbon atom, containing from 5 to 6 ring members with 1 heteroatom selected from sulfur and nitrogen, phenyl, a nitro substituted phenyl; R¹ is cyclopentyl; R² is a five- or six-membered heteroaromatic ring connected by a ring carbon atom to the amide group to the remainder of the compound, which heteroaromatic ring contains 1 or 2 heteroatoms selected from sulfur and nitrogen with a first heteroatom being nitrogen which is adjacent to the connecting ring carbon atom, said heteroaromatic ring being unsubstituted or monosubstituted at a position on a ring carbon atom other than adjacent to said connecting carbon atom with a substituent -(CH₂)ₙ-C(O)-OR⁷; n and y are independently 0 or 1; R⁴ and R⁵ are lower alkyl; R⁷ is hydrogen or lower alkyl and "lower" means 1 to 7 carbon atoms.

20. An amide of any of claims 1 to 19 selected from the group consisting of:
N- {4-[2-cyclopentyl-1-(thiazol-2-ylcarbamoyl)-ethyl]-pheny}4-nitrobenzamide,
N-{4-[2-cyclopentyl-1-(thiazol-2-ylcarbamoyl)-ethyl]-phenyl}-benzamide,
3-cyclopentyl-N-thiazol-2-yl-2-[4-(2-thiophen-2-yl-acetylamino)-phenyl]-propionamide,
N-{4-[2-cyclopentyl-1-(thiaxol-2-ylcarbamoyl)-ethyl]-phenyl}-isonicotinamide,
2-(3-cyclopentyl-2-{4-[(pyridine-3-carbonyl)-amino]-phenyl}-propionylamino)-thiazole-4-carboxylic acid ethyl ester,
N-{4-[2-cyclopentyl-1-(thiazol-2-ylcarbamoyl)-ethyl]-phenyl}-nicotinamide,
[2-(3-cyclopentyl-2-{4-[(pyridine-3-carbonyl)-amino]-phenyl}-propionylamino)-thiazol-4-yl]-acetic acid ethyl ester,
N-{4-[2-cydopenty)-1-(pyridin-2-ylcarbamoyl)-ethyl]-phenyl}-nicotinamide,
6-{3-cyclopentyl-2-{4-[(pyridine-3-carbonyl)-amino]-phenyl}-propionylamino)-nicotinic acid methyl ester,
6-(3-cyclopentyl-2-{4-[4-(pyridine-3-carbonyl)-amino]-phenyl}-propionylamino)-nicotinic acid,
N-{4-[2-cyclopentyl-1-(thiazol-2-ylcarbamoyl)-ethyl]-phenyl}-oxalamic acid methyl ester,
acetic acid {4-[2-cyclopentyl-1-(thiazol-2-ylcarbamoyl)-ethyl]-phenylcarbamoyl}-methyl ester,
3-cyclopentyl-2-(4-methanesulfonylamino-phenyl)-N-thiazol-2-yl-propionamide,
3-cyclopentyl-N-thiazol-2-yl-2-(4-trifluoromethanesulfonylamino-phenyl)-propionamide,
3-cyclopentyl-N-thiazol-2-yl-2-[4-(2,2,2-trifluoro-ethanesulfonylamino)-phenyl]-propionamide,
N-{4-[2-cyclopentyl-1-(thiazol-2-ylcarbamoyl)-ethyl]-phenyl}-dimethylsulfamide, and
3-cyclopentyl-2-[4-(4-nitro-benzenesulfonylamino)-phenyl]-N-thiazol-2-yl-propionamide.

21. A pharmaceutical composition comprising a compound of any of claims I to 20 and a pharmaceutically acceptable carrier and/or adjuvant.

22. A process for the preparation of a pharmaceutical composition of claim 21 comprising combining a compound of formula I according to any one of claims 1 to 20 with a pharmaceutically acceptable carrier and/or adjuvant.

23. The compounds according to any of claims 1 to 20 for use as a therapeutic active substance.

24. Compounds according to any of claims 1 to 20 for use in the treatment or prophylaxis of type II diabetes.

25. The use of a compound according to any of claims 1 to 20 for the preparation of a medicament for the treatment of prophylaxis of type II diabetes.

26. A process for the preparation of a compound of formula IA according to claim 2, which process comprises reaction of a compound of the formula VIII: 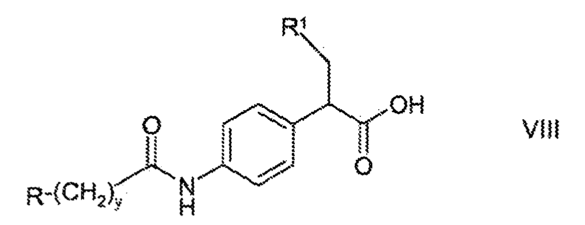 wherein R, R¹ and y are As defined in claim 1
with a primary amine of formula X:
R²-NH₂ X
wherein R² is as defined in claim 1;
to produce a compound of formula IA: 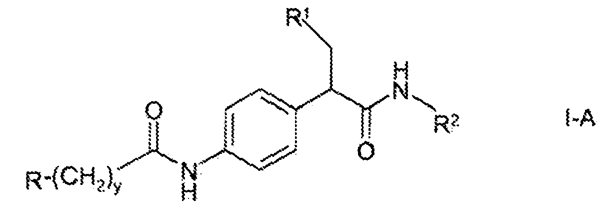 wherein R, R¹, R² and y are as defined in claim 1.

27. A process for the preparation of a compound of formula IB according to claim 3, which process comprises reaction of a compound of the formula XII: 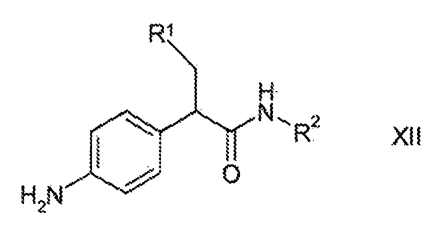 wherein R¹ and R² are as defined in claim 1;
with a sulfonyl chloride of formula XV: 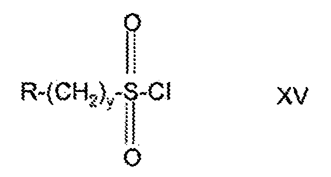 wherein R and y are as defined in claim 1;
to produce a compound of formula IB: 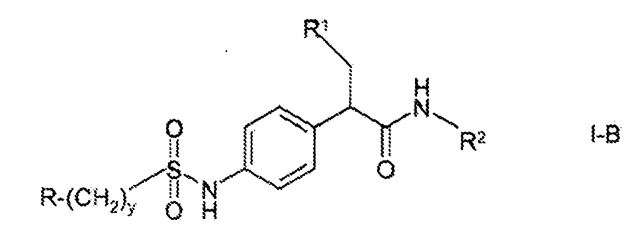 wherein R, R¹, R² and y are as defined in claim 1.

## Patentansprüche

1. Amid, ausgewählt aus der Gruppe, bestehend aus einer Verbindung der Formel 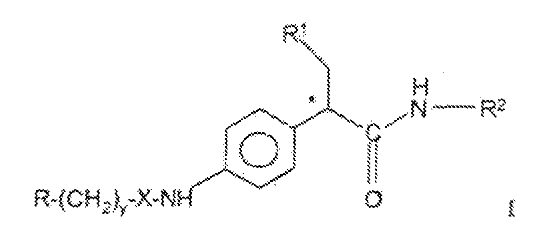 worin
X 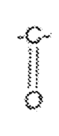 oder 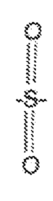 ist:
R Perfluor-niederalkyl, Niederalkyl, 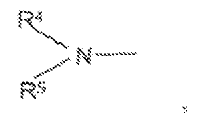 Niederalkoxycarbonyl, ein heteroaromatischer Ring, der durch ein Ringkohlenstoffatom gebunden ist, enthaltend 5 bis 6 Ringglieder mit 1 bis 3 Heteroatomen, ausgewählt aus der Gruppe, bestehend aus Sauerstoff, Schwefel und Stickstoff, unsubstituiertes Aryl, enthaltend 6 oder 10 Ringkohlenstoffatome, ein Nitro- oder Niederalkyl-substituiertes Aryl, wobei Aryl 6 oder 10 Ringkohlenstoffatome enthält, oder ein gesättigter 5-oder 6 gliedriger Cycloheteroalkylring, der durch ein Ringkohlenstoffatom gebunden ist, enthaltend 1 bis 2 Heteroatome, ausgewählt aus der Gruppe, bestehend aus Sauerstoff, Stickstoff, Schwefel, oder Cycloalkyl mit oder 6 Kohlenstoffatomen ist;
R¹ Cycloalkyl mit 5 oder 6 Kohlenstoffatomen ist;
R² ein fünf- oder sechsgliedriger heteroaromatischer Ring ist, der durch ein Ringkohlenstoffatom an die Amidgruppe an den Rest der Verbindung gebunden ist, wobei der heteroaromatische Ring 1. bis 3 Heteroatome enthält, ausgewählt aus der Gruppe, bestehend aus Sauerstoff, Schwefel und Stickstoff, wobei ein erstes Heteroatom Stickstoff ist, der zu dem Verbindungsringkohlenstoffatom nachbarständig ist, wobei der heteroaromatische Ring unsubstituiert eder an einer Stellung an einem anderen Ringkohlenstoffatom als dem zu dem Verbindungskoblenstoffatom nachbarständigen mit einem Substituenten, ausgewählt aus Niederalkyl und 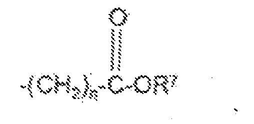 monosubstituiert ist,
n und y unabhängig voneinander eins ganze Zahl von 0 bis 4 sind;
R⁴ R⁵ und R⁷ unabhängig voneinander Wassertoff oder Niederalkyl sind; und
* das asymmetrische Kohlenstoffatom darstellt,
und wobei "nieder" 1 bis 7 Kohlenstoffatome bedeutet,
und einem pharmazeutisch akzeptablen Salz davon.

2. Amid nach Anspruch 1, wobei die Verbindung 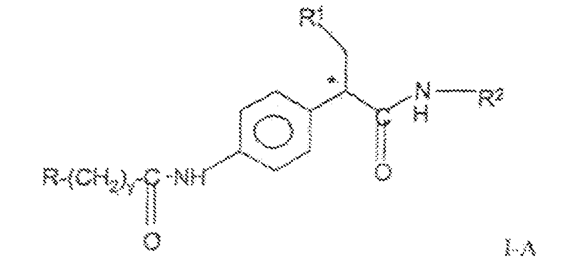 ist, worin R, R¹, R² und y wie in Anspruch 1 definiert sind.

3. Amid nach Anspruch 1, wobei die Verbindung 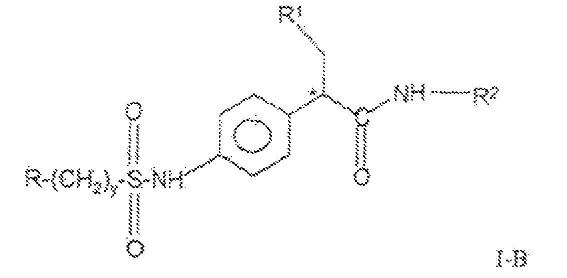 ist, worin R, R¹, R² und y wie in Anspruch 1 definiert sind.

4. Amid nach einem der Anspruche 1 bis 3, wobei R¹ Cyclopentyl ist.

5. Amid nach einem der Ansprüche 1 bis 4, wobei R² ein fünfgliedriger heteroaromatischer Ring ist, der durch ein Ringkohlenstoffatom an die Amidgruppe an den Rest der Verbindung gebunden ist, wobei der heteroaromatische Ring 1 bis 3 Heteroatome enthält, ausgewählt aus der Gruppe, bestehend aus Sauerstoff, Schwefel und Stickstoff, wobei ein erstes Heteroatom Stickstoff ist, der zu dem Verbindungsringkohlenstoffatom nachbarständig ist, wobei der heteroaromatische Ring unsubstituiert oder an einer Stellung an einem anderen Ringkohlenstoffatom als dem zu dem Verbindungskohlenstoffatom nachbarständigen mit einem Substituenten, ausgewählt aus Niederalkyl und -(CH₂)ₙ-C(O)OR⁷, monosubsituiert ist, und "nieder", n und R⁷ wie in Anspruch 1 definiert sind.

6. Amid nach einem der Ansprüche 1 bis 4, wobei R² ein sechsgliedriger heteroaromatischer Ring ist, der durch ein Ringkohlenstoffatom an die Amidgruppe an den Rest der Verbindung gebunden ist, wobei der heteroaromatische Ring 1 bis 3 Heteroatome enthält, ausgewählt aus der Gruppe, begehend aus Sauerstoff, Schwefel und Stickstoff, wobei ein erstes Heteroatom Stickstoff ist, der zu dem Verbindungsringkohlenstoffatom nachbarständig ist, wobei der heteroaromatische Ring unsubstituiert oder an einer Stellung an einem anderen Ringkohlenstoffatom als dem zu dem Verbindungskohlenstoffatom nachbarständigen mit einem substituenten, ausgewählt aus Niederalkyl und -(CH₂)ₙ-C(O)OR⁷, monosubsituiert ist; und "nieder", n und R⁷ wie in Anspruche 1 definiert sind.

7. Amid nach einem der Ansprüche 1 1 bis 4, wobei R² Thiazolyl ist, das durch ein Ringkohlenstoffatom an die Amidgruppe an den Rest der Verbindung gebunden ist, wobei Thiazolyl gegebenenfalls an einer Stellung an einem anderen Ringkohlenstoffatom als dem zu dem Verbindungskohlenstoffatom nachbarständigen mit einem Substituenten, ausgewählt aus Niederalkyl oder -(CH₂)ₙ-C(O)OR⁷, monosubstituiert ist; und "nieder", n und R⁷ wie in Anspruch 1 definiert sind.

8. Amid nach einem der Ansprüche 1 bis 4, wobei R² Pyridyl ist, das durch ein Ringkohlenstoffatom an die Amidgruppe an den Rest der Verbindung gebunden ist, wobei Pyridyl gegebenenfalls an einer Stellung an einem anderen Ringkohlenstoffatom als dem zu dem Verbindungskohlenstoffatom nachbarständigen mit einem Substituenten, ausgewählt aus Niederalkyl oder -(CH₂)ₙ-C(O)OR⁷, monosubstituiert ist; und "nieder", n und R⁷ wie in Anspruch 1 definiert sind.

9. Amid nach einem der Ansprüche 1 bis 8, wobei R Phenyl, Naphthyl oder Nitro-substituiertes Phenyl oder Naphthyl ist.

10. Amid nach einem der Ansprüche 1 bis 8, wobei R Nitro-substituiertes Phenyl ist.

11. Amid nach einem der Ansprüche 1 bis 8, wobei R unsubstituiertes Phenyl ist.

12. Amid nach einem der Ansprüche 1 bis 8, wobei R. ein heteroaromatischer Ring ist, der durch ein Ringkohlenstoffatom gebunden ist, enthaltend 5 bis 6 Ringglieder mit 1 bis 3 Heteroatomen, ausgewählt aus der Gruppe, bestehend aus Sauerstoff, Schwefel und Stickstoff.

13. Amid nach einem der Ansprüche 1 bis 8, wobei R Thienyl oder Pyridyl ist.

14. Amid nach einem der Ansprüche 1 bis 8, wobei R Niederalkoxycarbonyl ist und "nieder" 1 bis 7 Kohlenstoffatome bedeutet.

15. Amid nach einem der Ansprüche 1 bis 8, wobei R Niederalkyl oder Perfluorniederalkyl ist und "nieder" 1 bis 7 Kohlenstoffatome bedeutet.

16. Amid nach einem der Ansprüche 1 bis 8, wobei R -N(R⁴,R⁵) ist und R⁴ und R⁵ wie in Anspruch 1 definiert sind.

17. Amid nach einem der Ansprüche 1 bis 16, wobei n und y unabhängig voneinander 0 oder 1 sind.

18. Amid nach einem der Ansprüche 1 bis 17, wobei R⁴ und R⁵ Niederalkyl sind und nieder" 1 bis 7 Kohlenstoffatome bedeutet.

19. Amid nach einem der Ansprüche 1 bis 17, wobei X -C(O)- oder -S(O)₂- ist; R Perfluorniederalkyl, Niederalkyl, -N(R⁴,R⁵), Niederalkoxycarbonyl, ein heteroaromatischer Ring, der durch ein Ringkohlenstoffatom gebunden ist, enthaltend. 15 bis 6 Ringglieder, mit 1 Heteroatom, ausgewählt aus Schwefel und Stickstoff, Phenyl, ein Nitro-substituiertes Phenyl ist; R¹ Cyclopentyl ist; R² fünf- oder sechsgliedriger heteroaromatischer Ring ist, der durch ein Ringkohlenstoffatom an die Amidgruppe an den Rest der Verbindung gebunden ist, wobei der heteroaromatische Ring 1 oder 2 Heteroatome enthält, ausgewählt aus Schwefel und Stickstoff, wobei ein erstes Heteroatom Stickstoff ist, der zu dem Verbindungsringkohlenstoffatom nachbarständig ist, wobei der heteroaromatische Ring unsubstituiert oder an einer Stellung an einem anderen Ringkohlenstoffatom als dem zu dem Verbindungskohlenstoffatom nachbarständigen mit einem Substituenten -(CH₂)ₙ-C(O)-OR⁷ monosubstituiert ist; n und y unabhängig voneinander 0 oder 1 sind; R⁴ und R⁵ Niederalkyl sind; R⁷ Wasserstoff oder Niederalkyl ist und "nieder" 1 bis 7 Kohlenstoffatome bedeutet.

20. Amid nach einem der Ansprüche 1 bis 19, ausgewählt aus der Gruppe, bestehend aus
N-{4-[2-Cyclopentyl-1-(thiazol-2-ylcarbamoyl-ethy]-phenyl}-4-nitro-benzamid,
N-{4-[2-Cyclopentyl-1-(thiazol-2-ylcarbamoyl)-ethyl]-phenyl}-bezamid.
3-Cyclopentyl-N-thiazol-2-yl-2-[4-(2-thiophen-2-yl-acetylamino)-phenyl]-propionamid,
N-{4-[2-Cyclopentyl-1-(thiazol-2-ylcarbamoyl)-ethyl]-phenyl}-isonicotinamid,
2-(3-Cyclopentyl-2-{4-[(pyridin-3-carbonyl)-amino]-phenyl}-propionylamino)-thiazol-4-carbonsäureethylester,
N-{4-[2-Cyclopentyl-1-(thiazol-2-ylcarbamoyl)-ethyl]-phenyl}-nicotinamid,
[2-(3-Cyclopentyl-2-{4-[(pyridin-3-carbonyl)-amino]-phenyl}-phenyl}--propionylamino)-thiazol-4-yl]-essigsäureethylester,
N-{4-[2-Cyclopentyl-1-(pyridin-2-ylcarbamoyl)-ethyl]-phenyl}-nicotinamid,
6-(3-Cyclopentyl-2-{4-[(pyridin-3-carbonyl)-amino]-phenyl}-propionylamino)-nicotinsäuremethylester,
6-(3-Cyclopentyl-2-{4-[(pyridin-3-carbonyl)-phenil}-propionylamino)-nicotinsäure
N-{4-[2-Cyclopentyl-1-(thiazol-2-ylcarbamoyl)-ethyl]-phenyl}-carbamoylameisensäuremethylester,
Essigsäure{4-[2-cyclopentyl-1-(thiazol-2-ylcarbamoyl)-ethyl]-phenylcarbamoyl}-methylester,
3-Cyclopentyl-2-(4-methansulfonylamino-phenyl)-N-thiazol-2-yl-propionamid,
3-Cyclopentyl-N-thiazol-2-yl-2-(4-trifluormethansulfonylamino-phenyl)-propianamid,
3-Cyclopentyl-N-thiazol-2-yl-2-[4-(2,2,2-trifluor-ethansulfonylamino)-phenyl]-propionamid,
N-{4-[2-Cyclopentyl-1-(thiazol-2-ylcarbamoyl)-ethyl]-phenyl}-dimethylsulfamid und
3-Cyclopentyl-2-[4-(4-nitro-benzolsulfonylamino)-phenyl]-N-thiazol-2-ylpropionamid.

21. Pharmazeutische Zusammensetzung, umfassend eine Verbindung nach einem der Ansprüche 1 bis 20 und einen pharmazeutisch akzeptablen Träger und/oder ein pharmazeutisch akzeptables Adjuvans.

22. Verfahren zur Herstellung einer pharmazeutischen Zusammensetzung nach Anspruch 21, umfassend das Kombinieren einer Verbindung der Formel I nach einem der Ansprüche bis 20 mit einem pharmazeutisch akzeptablen Träger und/oder einem pharmazeutisch akzeptablen Adjuvans.

23. Verbindungen nach einem der Ansprüche 1 bis 20 zur Verwendung als eine therapeutisch aktive Substanz.

24. Verbindungen nach einem der Ansprüche 1 bis 20 zur Verwendung bei der Bebandlung oder Prophylaxe: von Diabetes Typ II.

25. Verwendung einer Verbindung nach einem der Ansprüche 1 bis 20 zur Herstellung eines Medikaments für die Behandlung oder Prophylaxe von Diabetes Typ II.

26. Verfahren zur Herstellung einer Verbindung der Formel IA nach Anspruch 2, wobei das Verfahren die Umsetzung einer Verbindung der Formel VIII: 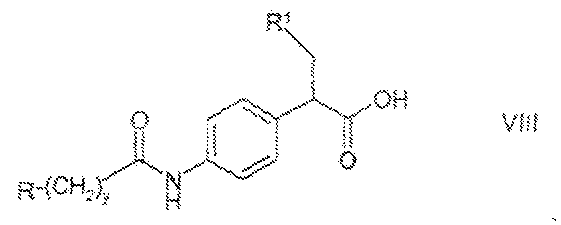 worin R, R¹ und y wie in Anspruch 1 definiert sind,
mit einem primären Amin der Formel X:
R²-NH₂ X,
worin R² wie in Anspruch 1 definiert ist,
zur Herstellung einer Verbindung der Formel 1A: 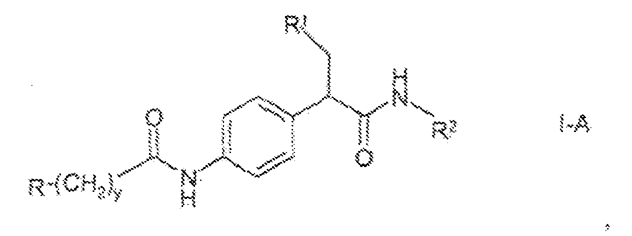 worin R, R¹, R² und y wie in Anspruch 1 definiert sind, umfaßt.

27. Verfahren zur Herstellung einer Verbindung der Formel IB nach Anspruch 3, wobei das Verfahren die Umsetzung einer der Verbindung der Formel XII: 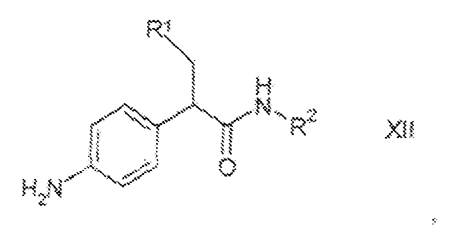 worin R¹ und R² wie in Anspruch definiert sind;
mit einem Sulfonylchlorid der Formel XV: 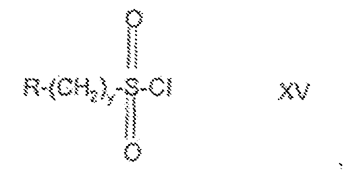 worin R und y wie in Anspruch 1 definiert sind;
zur Herstellung einer Verbindung der Formel IB: 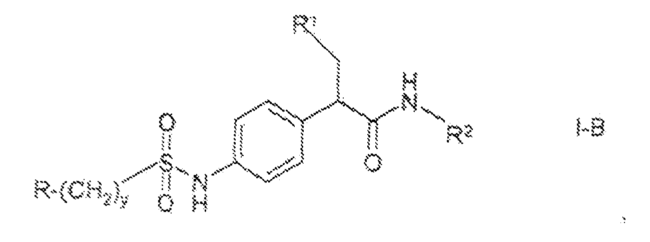 worin R, R¹, R² und y wie in Anspruch 1 definiert sind, umfaßt.

## Revendications

1. Amide choisi dans le groupe consistant en un composé de formule 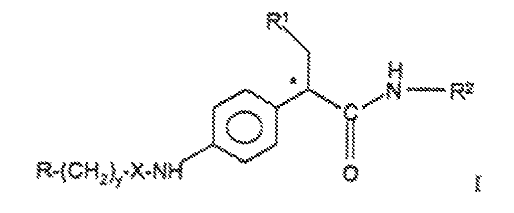 dans laquelle
X représente un groupe 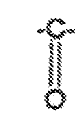 ou 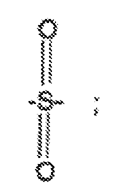 R représente un groupe perfluoralkyle inférieur, alkyle inférieur, 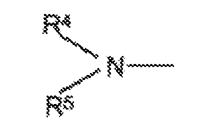 alkoxycarbonyle inférieur, un noyau hétéroaromatique, connecté par un atome de carbone du noyau, contenant 5 ou 6 membres dans le noyau avec 1 à 3 hétéroatomes choisis dans le groupe consistant en l'oxygène, le soufre et l'azote, un groupe aryle non substitué contenant 6 ou 10 atomes de carbone dans le noyau, un groupe aryle à substituant nitro ou alkyle inférieur, le groupe aryle contenant 6 ou 10 atomes de carbone dans le noyau ou un noyau hétérocycloalkyle penta- ou hexagonal saturé, connecté par un atome de carbone du noyau, contenant 1 ou 2 hétéroatomes choisis dans le groupe consistant en l'oxygène, l'azote et le soufre, ou un groupe cycloalkyle ayant 5 ou 6 atomes de carbone;
R² représente un groupe cycloalkyle ayant 5 ou 6 atome de carbone ;
R² représente un noyau hétéroaromatique penta- ou hexagonal connecté par un atome de carbone du noyau au groupe amide au reste du composé, un noyau hétéroaromatique qui contient 1 à 3 hétéroatomes choisis dans le groupe consistant en l'oxygène, le soufre et l'azote, un premier hétéroatome étant un atome d'azote qui est adjacent à l'atome de carbone de noyau de connexion, ledit noyau hétéroaromatique étant non substitué ou monosubstitué à une position sur un atome de carbone du noyau autre que la position adjacente audit atome de carbone de connexion avec un substituant choisi entre un groupe alkyle inférieur et un groupe 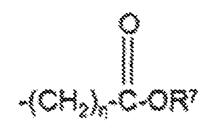 n et y représentent indépendamment un nombre entier de 0 à 4 ;
R⁴, R⁵ et R⁷ représentent indépendamment un atome d'hydrogène ou un groupe alkyle inférieur; et
* désigne l'atome de carbone asymétrique ;
et dans laquelle le terme "inférieur'' signifie 1 à 7 atomes de carbone ;
et un de ses sels pharmaceutiquement acceptables.

2. Amide suivant la revendication 1, ledit composé étant un composé de formule 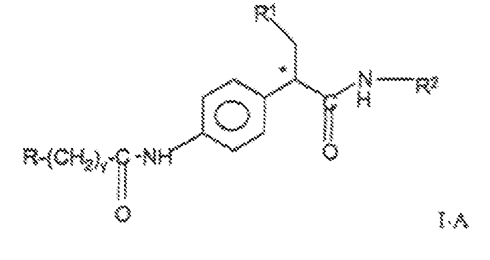 dans laquelle R, R¹, R² et y sont tels que définis dans la revendication 1.

3. Amide suivant la revendication 1, ledit composé étant un composé de formule 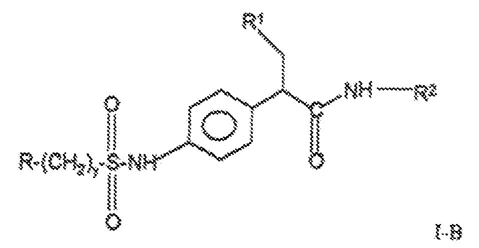 dans laquelle R, R¹, R² et y sont tels que définis dans la revendication 1.

4. Amide suivant l'une quelconque des revendications 1 à 3, dans lequel R¹ représente un groupe cyclopentyle.

5. Amide suivant l'une quelconque des revendications 1 à 4, dans lequel R² représente un noyau hétéroaromatique pentagonal connecté par un atome de carbone du noyau au groupe amide au reste du composé, le noyau hétéroaromatique contenant 1 à 3 hétéroatomes choisis dans le groupe consistant en l'oxygène, le soufre et l'azote, un premier hétéroatome étant un atome d'azote qui est adjacent à l'atome de carbone de noyau de connexion, ledit noyau hétéroaromatique étant non substitué ou monosubstitué à une position adjacente audit atome de carbone de connexion avec un substituant choisi entre un groupe alkyle inférieur et un groupe -(CH₂)ₙ-C(O)OR⁷; et "inférieur", n et R⁷ sont tels que définis dans la revendication 1.

6. Amide suivant l'une quelconque des revendications 1 à 4, dans lequel R² représente un noyau hétéroaromatique hexagonal connecté par un atome de carbone de noyau au groupe amide au reste du composé, le noyau hétéroaromatique contenant 1 à 3 hétéroatomes choisis dans le groupe consistant en l'oxygène, le soufre et l'azote, un premier hétéroatome étant un atome d'azote qui est adjacent à l'atome de carbone de noyau de connexion, ledit noyau hétéroaromatique étant non substitué ou monosubstitué à une position sur un atome de carbone de noyau autre que la position adjacente audit atome de carbone de connexion avec un substituant choisi entre un groupe alkyle inférieur et un groupe -(CH₂)ₙ-C(O)OR⁷ et "inférieur", n et R⁷ sont tels que définis dans la revendication 1.

7. Amide suivant l'une quelconque des revendications 1 à 4, dans lequel R² représente un groupe thiazolyle connecté par un atome de carbone de noyau au groupe amide au reste du composé, ledit groupe thiazolyle étant facultativement monosubstitué a une position sur un atome de carbone de noyau autre que la position adjacente audit atome de carbone de connexion avec un substituant choisi entre un groupe alkyle inférieur et un groupe -(CH₂)ₙ-C(O)OR⁷ ; et "inférieur", n et R⁷ sont tels que définis dans la revendications 1.

8. Amide suivant l'une quelconque des revendications 1 à 4, dans lequel R² représente un groupe pyridyle connecté par un atome de carbone de noyau au groupe amide au reste du composé, ledit groupe pyridyle étant facultativement monosubstitué à une position sur un atome de carbone de noyau autre que la position adjacente audit atome de carbone de connexion avec un substituant choisi entre un groupe alkyle inférieur et un groupe -(CH₂)ₙ-C(O)OR⁷ ; et "inférieur", n et R⁷ sont tels que définis dans la revendication 1.

9. Amide suivant l'une quelconque des revendications 1. à 8, dans lequel R représente un groupe phényle, un groupe naphtyle ou un groupe phényle ou naphtyle à substituant nitro.

10. Amide suivant l'une quelconque des revendications 1 à 8, dans lequel R représente un groupe phényle à substituant nitro.

11. Amide suivant l'une quelconque des revendications 1. à 8, dans lequel R représente un groupe phényle non substitué.

12. Amide suivant l'une quelconque des revendications 1 à 8, dans lequel R représente un noyau hétéroaromatique, connecté par un atome de carbone de noyau, contenant 5 ou 6 membres de noyau avec 1 à 3 hétéroatomes choisis dans le groupe consistant en l'oxygène, le soufre et l'azote.

13. Amide suivant l'une quelconque des revendications 1 à 8, dans lequel R représente un groupe thiényle ou pyridyle.

14. Amide suivant l'une quelconque des revendications 1 à 8, dans lequel R représente un groupe (alkoxy inférieur) carbonyle, et "inférieur" signifie 1 à 7 atomes de carbone.

15. Amide suivant l'une quelconque des revendications 1 à 8, dans lequel R représente un groupe inférieur ou perfluoralkyle inférieur, et "inférieur" signifie 1 à 7 atomes de carbone.

16. Amide suivant l'une quelconque des revendications 1 à 8, dans lequel R représente un groupe -N(R⁴,R⁵) et R⁴ et R⁵ sont tels que définis dans la revendication 1.

17. Amide suivant l'une quelconque des revendications 1 à 16, dans lequel n et y sont égaux indépendamment à 0 ou 1.

18. Amide suivant l'une quelconque des revendications 1 à 17, dans lequel R⁴ et R⁵ représentent des groupes alkyle inférieur, et "inférieur" signifie 1 à 7 atomes de carbone.

19. Amide suivant l'une quelconque des revendications 1 à 17, dans lequel X représente un groupe -C(O)- ou -S(O)₂-, R représente un groupe perfluoralkyle inférieur, alkyle inférieur, -N(R⁶,R⁵), (alkoxy inférieur) carbonyle, un noyau hétéroaromatique, connecté par un atome de carbone de noyau, contenant 5 ou 6 membres dans le noyau avec un hétéroatome choisi entre le soufre et l'azote, un groupe phényle, un groupe phényle à substituant nitro ; R¹ représente un groupe cyclopentyle ; R² représente un noyau hétéroaromatique penta- ou hexagonal connecté par un atome de carbone de noyau au groupe amide au reste du composé, le noyau hétéroaromatique contenant 1 ou 2 hétéroatomes choisis entre le soufre et l'azote un premier hétéroatome étant un atome d'azote qui est adjacent à l'atome de carbone de noyau de connexion, ledit noyau hétéroaromatique étant non substitué ou monosubstitué à une position sur un atome de carbone de noyau autre que la position adjacente audit atome de carbone de connexion avec un substituant -(CH₂)ₙ-C(O)-OR⁷ ; n et y sont égaux indépendamment à 0 ou 1 ; R⁶ et R⁵ représentent des groupes alkyle inférieur ; R⁷ représente un atome d'hydrogène ou un groupe alkyle inférieur; et "inférieur" signifie 1 à 7 atomes de carbone,

20. Amide suivant l'une quelconque des revendications 1 à 19, choisi dans le groupe consistant en ;
N-{4-[2-cyclopentyl-1-(thiazole-2-ylcarbamoyl)-éthyl]-phényl}-4-nitro-benzamie,
N-{4-[2-cyclopentyl-1-(thiazole-2-ylcarbamoyl)-éthyl]-phényl}-benzamide,
3-cyclopentyl-N-thiazole-2-yl-2-[4-(2-thiophène-2-yl-acétylamino)-phényl]-propionamide,
N-{4-[2-cyclopentyl-1-(thiazole-2-ylcarbamoyl)-éthyl]-phényl}-isonicotinamide,
ester éthylique d'acide 2-(3-cyclopentyl-2-{4-[{pyridine-3-carbonyl)-amino]-phényl}-propionylamino)-thiazole-4-carboxylique,
N-{4-[2-cyclopentyl-1-(thiazole-2-ylcarbamoyl)-éthyl]-phényl}-nicotinamide,
ester éthylique d'acide [2-{3-cyclopentyl-2-{4-[(pyridine-3-carbonyl)-amino]-phényl}- propionylamino}-thiazole-4-yl]-acétique,
N-{4-[2-cyclopentyl-1-(pyridine-2-ylcarbamoyl)-éthyl]-phényl}-nicotinamide,
ester méthylique d'acide 6-(3-cyclopentyl-2-{4-[(pyridine-3-carbonyl)-amino]-phényl}-propionylamino)-nicotinique,
acide 6-(3-cyclopentyl-2-{4-[(pyridine-3-carbonyl)-amino]-phényl}-propinylamino)-nicotinique,
ester méthylique d'acide N-{4-[2-cyclopentyl-1-(thiazole-2-ylcarbamoyl)-éthyl]-phényl}oxelamique,
ester {4-[2-cyclopentyl-1-(thiazole-2-ylcarbamoyl)-éthyl]-phénylcarbamoyl}-méthylique d'acide acétique,
3-cyclopentyl-2-(4-méthanesulfonylamino-phényl)-N-thiazole-2-yl-propionamide,
3-cyclopentyl-N-thiazole-2-yl-2-(4-trifluorométhane-sulfonylamino-phényl)-propionamide,
3-cyclopentyl-N-thiazole-2-yl-2-[4-(2,2,2-trifluoroéthanesulfonylamino-phenyl]propionamide,
N-{-[2-cyclopentyl-1-(thiazole-2-ylcarbamoyl)-éthyl]-phényl}-diméthylsulfamide, et
3-cyclopentyl-2-{4-(4-nitro-benzènesulfonylamino)-phènyl]-N-thiazole-2-yl-propionamide.

21. Composition pharmaceutique comprenant un composé de l'une quelconque des revendications 1 à 20 et un support et/ou adjuvant pharmaceutiquement acceptable.

22. Procédé pour la préparation d'une composition pharmaceutique de la revendication 21, comprenant la combinaison d'un composé de formule I suivant l'une quelconque des revendications 1 à 20 avec un support et/ou adjuvant pharmaceutiquement acceptable.

23. Composés suivant l'une quelconque des revendications 1 à 20, destinés à être utilisés comme substance thérapeutique active.

24. Composés suivant l'une quelconque des revendications 1 à 20, destinés à être utilisés dans le traitement ou la prophylaxie du diabète de type II.

25. Utilisation d'un composé suivant l'une quelconque des revendications 1 à 20, pour la préparation d'un médicament destiné au traitement ou à la prophylaxie du diabète de type II.

26. Procédé pour la préparation d'un composé de formule IA suivant la revendication 2, procédé qui comprend la réaction d'un composé de formule VIII : 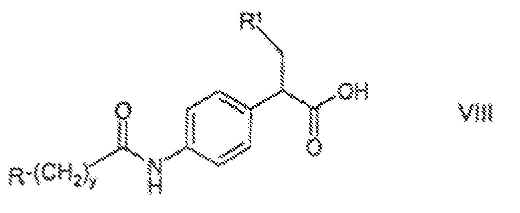 dans laquelle R, R¹ et y sont tels que définis dans la revendication 1 ;
avec une aminé primaire de formule X :
**R²-NH₂** **X**
dans laquelle R² est tel que défini dans la revendication 1 ;
pour produira un composé de formule IA : 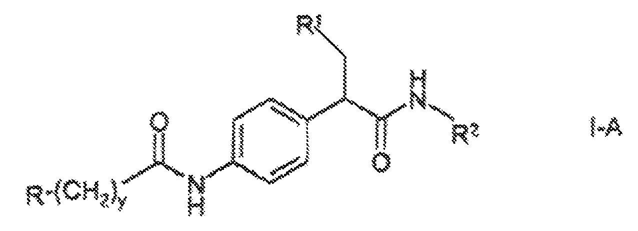 dans laquelle R, R¹, R² et y sont tels que définis dans la revendication 1.

27. Procédé pour la préparation d'un composé de formule IB suivant la revendication 3, procédé qui comprend la réaction d'un composé XII : 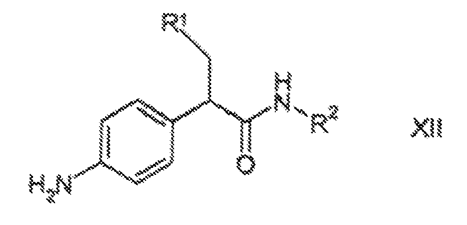 dans laquelle R¹ et R² sont tels que définis la revendication 1 ;
avec un chlorure de sulfonyle de formule XV : 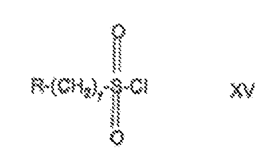 dans laquelle R et y sont tels que définis dans la revendication 1 ;
pour produire un composé de formule IB : 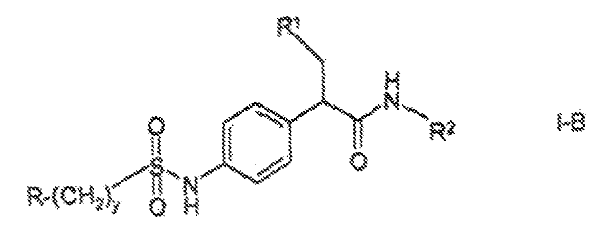 dans laquelle R, R¹, R² et y sont tels que définis dans la revendication 1.
